# EUROPEAN PATENT APPLICATION

(11) **EP 4 360 642 A1**
(43) Date of publication of application: **01.05.2024**
(21) Application number: 22827706.7
(22) Date of filing: 24.06.2022
(51) Int. Cl.: A61K 38/16, A61K 31/00, A61P 9/00, A61P 3/06

(54) **PHARMACEUTICAL COMPOSITION OF PCSK9 INHIBITOR AND GLP-1 RECEPTOR AGONIST**

(30) Priority: 25.06.2021 CN 202110712283
(71) Applicant: GAN & LEE PHARMACEUTICALS CO., LTD., Beijing 101109 (CN)
(72) Inventor: JIAO, Jiao, Beijing 101109 (CN); GAN, Zhongru, Beijing 101109 (CN); WANG, Zhen, Beijing 101109 (CN); CHEN, Wei, Beijing 101109 (CN); WANG, Xi, Beijing 101109 (CN); ZHANG, Yining, Beijing 101109 (CN)
(74) Representative: Kraus & Lederer PartGmbB
(86) International application number: PCT/CN2022/101291
(87) International publication number: WO 2022/268214

(57) **Abstract**

The present invention relates to a pharmaceutical combination comprising a PCSK9 inhibitor and a GLP-1 receptor agonist; The invention also relates to the use of the pharmaceutical combination in preparing drugs for treating hyperglycemia, diabetes, obesity and/or cholesterol related diseases.

## Description

### TECHNICAL FIELD

The present invention relates to the field of a composition, comprising a) a PCSK9 inhibitor, and b) a GLP-1 receptor agonist; and further relates to the use of said pharmaceutical composition in the preparation of a drug for the treatment of hyperglycemia, diabetes, obesity and/or cholesterol-related diseases.

### BACKGROUND

Hyperglycemia and hyperlipidemia are one of the most serious public health problems faced by all countries today, which are particularly evident in middle-aged and elderly people.

Cholesterol is an essential lipid substance for the human life activities. Elevated levels of plasma cholesterol can lead to atherosclerotic cardiovascular disease and greatly increase the risk of myocardial infarction and stroke. There are 60-70% of plasma cholesterol transported by low density lipoprotein (LDL) in human body, and about 75% of plasma LDL is cleared by endocytosis into liver cells via the low-density lipoprotein receptor (LDLR) in liver cells, the remaining 25% is absorbed and utilized by peripheral tissues and organs. The LDLR-mediated LDL endocytosis pathway is considered to be the most important and efficient way for the body to remove plasma LDL. Protein drugs currently marketed or under development for the alleviation or treatment of cholesterol-related symptoms include PCSK9 inhibitors (proprotein convertase subtilise kexin 9, PCSK9), ANGPTL 3 inhibitor (Angiopoietin- like 3), ANGPTL 4/8 inhibitor (Angiopoietin-like protein 4/8), Apoc3 inhibitor (apolipoprotein C3) and apolipoprotein (a) inhibitor (lipoprotein a).

PCSK9 (proprotein convertase subtilise kexin 9, PCSK9) protein will mediate the degradation of LDL receptor by binding to the LDL receptor on the surface of hepatocyte, at this time, the amount of LDL mediated by LDL receptor entering the hepatocyte for degradation is reduced, resulting in the higher level LDL in plasma. Currently, PCSK9 monoclonal antibody is a very good lipid-lowering drug, through binding to PCSK9 to mediate the degradation of PCSK9, thereby increasing the level of LDL receptors on the surface of hepatocytes to reduce the level of LDL in plasma, to achieve the effect of lowering blood lipids. The main PCSK9 antibodies currently on the market are Sanofi's Alirocumab, Amgen's Evolocumab, and other PCSK9 antibodies in the clinical stage include Lodelcizumab (Norvatis), Ralpancizumab (Pfizer) , Bococizumab (Pfizer) , LY3015014 <Eli Lilly) , LIB-003 (Adenexus Therapeutics Inc) , SHR-1209 < Suzhou ShengdiyaBiomedical Co Ltd) , AK-102 (Akeso BiopHarma Inc) , JS-002 < Shanghai Junshi Biosciences Co Ltd) , SAL-003 < Shenzhen Salubris pHarmaceuticals Co Ltd) , AK-102 (Akeeso BiopHarma Inc) , ATH-06 (AFFiRiS) and so on.

Currently, peptide drugs used for hypoglycemia mainly include GLP-1 (glucagon-like peptide-1, GLP-1) agonists , GIP (gastric inhibitory polypeptide, GIP) agonists, GCGR (glucagon receptor) agonists, FGF21 (Fibroblast growth factor 21) agonists, and agonists targeting any two or three of the GLP-1, GIP, GCGR, or FGF21 targets.

GLP-1 (Glucagon-Like Peptide-1, GLP-1) is secreted by gastrointestinal mucosal L-cells in response to meals. GLP-1 acts primarily through the GLP-1 receptor (GLP-1R) expressed on pancreatic islet β-cells and δ-cells (D-cells), and after GLP-1 binds to GLP-1R, it can mediate the secretion of growth inhibitor (SMS) from islet δ cells, and SMS in turn inhibits glucagon secretion from islet α cells via the growth inhibitor 2 receptor (SSTR2). On the other hand, GLP-1 mediates the increase of insulin synthesis and secretion through GLP-1R on β-cells, which acts to lower blood glucose through these two simultaneous effects. The main GLP-1 receptor agonists that have been marketed so far are exenatide, beinaglutide and liraglutide. The long-acting GLP-1 receptor agonists are PEG-Loxenatide, semaglutide, albiglutide, dulaglutide and so on.

According to the literature (refer to Cardiovascular safety and efficacy of the PCSK9 inhibitor evolocumab in patients with and without diabetes and the effect of evolocumab on glycaemia and risk of new-onset diabetes: a prespecified analysis of the FOURIER randomised controlled trial, Marc S Sabatine, Lawrence A Leiter, et al., Lancet Diabetes Endocrinol. Volume 5, Issue 12, December 2017, Pages 941-950) reports. About 40% of patients with hyperlipidemia also suffer from hyperglycemia; and about 40% of patients with hyperglycemia also have substandard low-density lipoprotein cholesterol (LDL-C) levels after taking statin for lipid-lowering therapy, therefore the number of patients who need to control both lipids and blood glucose at the same time is in a large amount.

In order to serve said populations with both hyperlipidemia and hyperglycemic, MedImmune has developed a PCSK9 monoclonal antibody and GLP-1 analog peptide fusion protein MEDI4166, with the aim that this fusion protein, MEDI4166, can achieve the lipid-lowering effect of PCSK9 monoclonal antibody and the glucose-lowering effect of GLP-1 analog peptide at the same time. However, when MEDI4166 entered the phase I clinical trials, MEDI4166 (highest dose 400 mg) only showed LDL-C lowering effect, but no glucose lowering effect was found. Therefore, there is an urgent need to develop a drug that can treat both hyperglycemia and hyperlipidemia.

### Summary

The present invention provides a pharmaceutical combination comprising PCSK9 inhibitors and GLP-1, which unexpectedly maintains GLP-1's hypoglycemic function or has good synergistic effects in lowering blood sugar while maintaining the preventive or therapeutic effects of PCSK9 inhibitors on cholesterol related symptoms. The pharmaceutical composition of the present invention has excellent effects on lowering blood sugar, reducing body weight, and alleviating cholesterol related symptoms.

The first aspect of the present invention provides a pharmaceutical combination comprising:
a) a protein drug for alleviating or treating cholesterol-related symptoms, preferably, the protein drug is selected from a PCSK9 inhibitor, an ANGPTL3 inhibitor, an ANGPTL4/8 inhibitor, an Apoc3 inhibitor and apolipoprotein(a) inhibitor, and further preferably, the protein drug is a PCSK9 inhibitor; and
b) a hypoglycemic polypeptide drug, preferably, the polypeptide drug is selected from a GLP-1 receptor agonist, a GIP receptor agonist, a GCCR receptor agonist, a FGF21 agonist and an agonist targeting any two or three targets of GLP-1R, GIPR, GCCR or FGF21R. Further preferably, the polypeptide drug is a GLP-1 receptor agonist.

Optionally, the PCSK9 inhibitor is an antibody or an antigen-binding fragment thereof that specifically binds to PCSK9.

Optionally, the PCSK9 inhibitor is selected from at least one from the group consisting of alirocumab, evolocumab, Lodelcizumab, Ralpancizumab, Bococizumab, LY3015014, LIB-003, SHR-1209, AK-102, JS-002, SAL-003, AK-102 and ATH-06; preferably the PCSK9 inhibitor is alirocumab or evolocumab.

Optionally, the GLP-1 receptor agonist is selected from polyethylene glycol loxenatide, dulaglutide, semaglutide, albiglutide, N-ε²⁶-(17-Carboxyheptadecanoylamino)-4(S)-carboxybutanoyl-[Arg34]GLP-1-(7-37) peptide, N-ε²⁶-(17-Carboxyheptadecanoylamino)-4(S)-carboxybutanoyl-[Gly8,Arg34]GLP-1-(7-37) peptide, N-ε²⁶-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl][ Gly8,Arg34]GLP-1-(7-37) peptide, N-ε³⁰-[2-(2-[2-(2-[2-(2-[4-(17-Carboxyheptadecanoylamino)-4(S)-carboxybutanoylamino] ethoxy)ethoxy] acetamido)ethoxy]ethoxy)acetyl]( Val⁸ Glu²² Lys³⁰ Arg^{26,34}-GLP-1(7-37)) peptide, or N-ε²³-[2-(2-[2-(2-[2-(2-[4-(17-Carboxyheptadecanoylamino)-4(S)-carboxybutanoylamino]ethoxy)ethoxy]acetamido)ethoxy]ethoxy)acetyl]( Val⁸ Glu²² Lys²³ Arg^{26,34}-GLP-1(7-37)) peptide, or GLP-1 receptor agonist is a compound of formula B:

[Acy-(L1)ᵣ-(L2)_{q}]-G1 (B),

wherein G1 is a GLP-1 analogue having Arg and Ala or Gly respectively at positions corresponding to position 34 and position 8, respectively, of GLP-1(7-37) (SEQ ID NO: 1),, and [Acy-(L1)ᵣ-(L2)_{q}] is a substituent linked to an ε amino group of the Lys residue at position 26 of the GLP-1 analogue, wherein
r is an integer from 1 to 10, and q is 0 or an integer from 1 to 10;
Acy is a fatty diacid comprising 20-24 carbon atoms, wherein formally, a hydroxyl group has been removed from one of carboxyl groups in the fatty diacid;
L1 is an amino acid residue selected from the following: γGlu, αGlu, βAsp, αAsp, γ-D-Glu, α-D-Glu, β-D-Asp and α-D-Asp;
L2 is a neutral and alkylene glycol-containing amino acid residue;
Acy, L1 and L2 are linked by amide bonds; and the order of occurrence of L1 and L2 in the formula (B) can be independently interchanged.

Optionally, G1 is [Gly8, Arg34]GLP-1-(7-37) peptide(SEQ ID NO: 2) or [Arg34]GLP-1-(7-37) peptide(SEQ ID NO: 3), and preferably [Gly8, Arg34]GLP-1-(7-37) peptide; and/or
Optionally, r is 1, 2, 3, 4, 5 or 6; preferably, r is 1, 2, 3 or 4; preferably, r is 1 or 2; preferably, r is 1; and/or
Optionally, q is 0, 1, 2, 3, 4, 5, 6, 7 or 8; preferably, q is 0, 1, 2, 3 or 4; more preferably, q is 0, 1 or 2; and/or
Optionally, Acy is a fatty diacid containing 20-23 carbon atoms; preferably, Acy is a fatty diacid containing 20, 21 or 22 carbon atoms.
Optionally, L2 is -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably, L2 is -HN-(CH₂)₂-O-(CH₂)₂-O-CH₂-CO-; and/or
Optionally, L1 is selected from γGlu and βAsp; preferably, L1 is γGlu; and/or
Optionally, Acy is HOOC-(CH₂)₁₈-CO-, HOOC-(CH₂)₁₉-CO-, HOOC-(CH₂)₂₀-CO-, HOOC-(CH₂)₂₁-CO- or HOOC-(CH₂)₂₂-CO-; preferably, Acy is HOOC-(CH₂)₁₈-CO-, HOOC-(CH₂)₂₀-CO- or HOOC-(CH₂)₂₂-CO-. Optionally, the Acy, L1, and L2 in formula (B) are sequentially linked by amide bonds, and the C- terminal of L2 is linked to the ε amino group of the Lys residue at position 26 of the GLP-1 analogue.

Optionally, the GLP-1 receptor agonist is selected from the following compounds:
*N*-ε-²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl] [Arg34]GLP-1-(7-37) peptide,
*N*-ε²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1-(7-37) peptide;
preferably, the compound is selected from the following group consisting of:
   *N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
   *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
   *N*-ε²⁶-(19-carboxynonadecanoylamino)-4(*S*)-carboxybutanoyl-[Gly8, Arg34]GLP-1-(7-37) peptide,
   *N*-ε²⁶-(19-carboxynonadecanoylamino)-4(*S*)-carboxybutanoyl-[Arg34]GLP-1-(7-37) peptide,
   *N*-ε²⁶-[2-(2-[2-(2-[2-(2-[4-(21-carboxyheneicosanoylamino)-4(*S*)-carboxybutanoylamino] ethoxy)ethoxy] acetylamino)ethoxy]ethoxy)acetyl ][Gly8, Arg34]GLP-1-(7-37) peptide, and
   *N*-ε²⁶-[2-(2-[2-(4-[21-carboxyheneicosanoylamino]-4(*S*)-carboxybutanoylamino)ethoxy]ethoxy)acetyl] [Gly8, Arg34]GLP-1-(7-37) peptide.

Optionally, the PCSK9 inhibitor and the GLP-1 receptor agonist are administered simultaneously or separately.

Optionally, the combination is in the form of a pharmaceutical composition or kit.

Optionally, the pharmaceutical combination further comprising one or more pharmaceutically acceptable excipients, preferably, the pharmaceutically acceptable excipients are selected from at least one from the group consisting of buffer, stabilizer, surfactant, and isotonic agent. Optionally, the buffer is selected from at least one from the group consisting of citric acid, citrate, sodium acetate, sodium dihydrogen phosphate, glutamate, histidine, disodium hydrogen phosphate, and trishydroxymethylaminomethane buffer, preferably, the buffer is selected from at least one from the group consisting of citric acid, sodium acetate and disodium hydrogen phosphate; preferably, the buffer is a combination of citric acid and disodium hydrogen phosphate; and/or
the stabilizer is selected from at least one from the group consisting of polyhydroxy hydrocarbons, disaccharides, benzyl alcohol, amino acids, polyols, and phenol, preferably selected from sucrose, lactose, fructose, maltose, trehalose, sorbitol, mannitol, arginine, lysine, methionine, taurine and proline; preferably at least one ofhistidine, proline and sucrose; and/or
the surfactant is selected from at least one from the group consisting of polysorbate 80, polysorbate 20, and poloxamer 188; and/or the isotonic agent is selected from at least one from the group consisting of sodium chloride, propylene glycol, and glycerol, preferably propylene glycol.

Optionally, the content of the buffer in the combination is 0.05mM to about 100mM, preferably about 0.05mM to about 85mM, preferably about 0.05mM to about 82mM, preferably about 0.05mM to about 40mM, preferably about 0.1mM to about 30mM, Preferably about 1mM to about 20mM, preferably about 5mM to about 15mM, preferably about 5mM to about 10mM; and/or
the content of the stabilizer in the combination is about 0.1% w/v to about 15% w/v, preferably about 0.5% w/v to about 10% w/v, preferably about 2.5% w/v to about 10% w/v; and/or
the surfactant is presented in the combination in an amount from about 0.001% w/v to about 10% w/v, preferably from about 0.01% w/v to about 1% w/v; and/or
the pH of the pharmaceutical combination is about 4.0-about 8.0, preferably about 4.5-about 7.5, preferably about 4.5-about 7.0, preferably about 4.5-about 6.5, preferably about 5.6-about 6.2, preferably about 4.5-about 5.5, preferably about 5.0 to about 5.5.

For example, the pH of the pharmaceutical combination of the present invention may be about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5.

The second aspect of the present invention discloses a pharmaceutical composition, comprising a GLP-1 receptor agonist as defined in the first aspect; the PCSK9 inhibitor as defined in the first aspect; buffer, the buffer is selected from at least one from the group consisting of citric acid, citrate, sodium acetate, sodium dihydrogen phosphate, glutamate, disodium hydrogen phosphate, and trishydroxymethylaminomethane buffer, preferably, the buffer is selected from at least one from the group consisting of citric acid, sodium acetate and disodium hydrogen phosphate, preferably the buffering agent is a combination of citric acid and disodium hydrogen phosphate;
stabilizer, the stabilizer is selected from at least one from the group consisting of polyhydroxy hydrocarbons, disaccharides, benzyl alcohol, amino acids, polyols, and phenol, preferably, the stabilizer is selected from sucrose, lactose, fructose, maltose, trehalose, sorbitol, mannitol, arginine, lysine, methionine, taurine, histidine and proline; preferably, the stabilizer is selected from at least one from the group consisting of histidine, proline and sucrose; and
a surfactant, the surfactant is selected from at least one from the group consisting of polysorbate 80, polysorbate 20, and poloxamer 188. Optionally, the GLP-1 receptor agonist is selected from the following compounds: semaglutide,
N-ε²⁶-(17-Carboxyheptadecanoylamino)-4(S)-carboxybutanoyl-[Arg34]GLP-1-(7-37) peptide,
N-ε²⁶-(17-Carboxyheptadecanoylamino)-4(S)-carboxybutanoyl-[Gly8,Arg34]GLP-1-(7-37) peptide,
N-ε²⁶-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl][ Gly8,Arg34]GLP-1-(7-37) peptide,
N-ε³⁰-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val⁸ Glu²² Lys³⁰ Arg^{26,34}-GLP-1(7-37)) peptide,
N-ε²³-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val⁸ Glu²² Lys²³ Arg^{26,34}-GLP-1(7-37)) peptide,
N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide,
N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[21-Carboxynodecanoylamino]-4(S)-carboxybutanoylamino)ethoxy]ethoxy)acetyl][Gly8, Arg34 ]GLP-1-(7-37) peptide,
N-ε²⁶-[2-(2-[2-(2-[2-(2-[4-(23-Carboxytricanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[23-Carboxytricanoylamino]-4(S)-carboxybutanoylamino)ethoxy]ethoxy)acetyl][Gly8, Arg34 ]GLP-1-(7-37) peptide,
N-ε²⁶-(23-Carboxytricosylamino)-4(S)-carboxybutanoyl-[Gly8, Arg34]GLP-1-(7-37) peptide,
N-ε²⁶-(19-Carboxynonadecanoylamino)-4(S)-carboxybutanoyl-[Gly8, Arg34]GLP-1-(7-37) peptide,
N-ε²⁶-(21-Carboxynodecanoylamino)-4(S)-carboxybutanoyl-[Gly8, Arg34]GLP-1-(7-37) peptide,
N-ε²⁶-[2-(2-[2-(2-[2-(2-[4-(19-Carboxynonadenylamino)-4(S)-carboxybutanoylamino]ethoxy)ethoxy]acetylamino)ethoxy]ethoxy)acetyl ][Arg34]GLP-1-(7-37) peptide,
N-ε²⁶-[2-(2-[2-(4-[19-Carboxynonadecanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(21-carboxynodecanoylamino)-4(S)-carboxybutanoylamino]ethoxy)ethoxy [Arg34]GLP-1-(7-37) peptide,
N-ε²⁶-[2-(2-[2-(4-[21-Carboxynodecanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(23-Carboxytricanoylamino)-4(S)-carboxybutanoylamino]ethoxy)ethoxy [Arg34]GLP-1-(7-37) peptide,
N-ε²⁶-[2-(2-[2-(4-[23-Carboxytricosanoylamino]-4(S)-carboxybutanoylamino)ethoxy]ethoxy)acetyl][Arg34]GLP-1-(7-37)peptide,
N-ε²⁶-(23-Carboxytricosanoylamino)-4(S)-carboxybutanoyl-[Arg34]GLP-1-(7-37) peptide,
N-ε²⁶-(19-Carboxynonadecanoylamino)-4(S)-carboxybutanoyl-[Arg34]GLP-1-(7-37) peptide,
N-ε²⁶-(21-Carboxynodecanoylamino)-4(S)-carboxybutanoyl-[Arg34]GLP-1-(7-37) peptide,
N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[20-Carboxyicosanoylamino]-4(S)-carboxybutanoylamino)ethoxy]ethoxy)acetyl][Gly8, Arg34]GLP-1-(7-37) peptide,
N-ε²⁶-[2-(2-[2-(2-[2-(2-[4-(22-Carboxydodecanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[22-Carboxydodecanoylamino]-4(S)-carboxybutanoylamino)ethoxy]ethoxy)acetyl][Gly8, Arg34 ]GLP-1-(7-37) peptide,
N-ε²⁶-(20-Carboxyeicosanoylamino)-4(S)-carboxybutanoyl-[Gly8, Arg34]GLP-1-(7-37) peptide,
N-ε²⁶-(22-Carboxydodecanoylamino)-4(S)-carboxybutanoyl-[Gly8, Arg34]GLP-1-(7-37) peptide,
N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Arg34]GLP-1-(7-37) peptide,
N-ε²⁶-[2-(2-[2-(4-[20-Carboxyeicosanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(22-Carboxydodecanoylamino)-4(S)-carboxybutanoylamino]ethoxy)ethoxy [Arg34]GLP-1-(7-37) peptide,
N-ε²⁶-[2-(2-[2-(4-[22-Carboxydodecanoylamino]-4(S)-carboxybutanoylamino)ethoxy]ethoxy)acetyl][Arg34]GLP-1-(7-37)peptide,
N-ε²⁶-(20-Carboxyeicosanoylamino)-4(S)-carboxybutanoyl-[Arg34]GLP-1-(7-37) peptide, or
N-ε²⁶-(22-Carboxydocanoylamino)-4(S)-carboxybutanoyl-[Arg34]GLP-1-(7-37) peptide;
Preferably, the compound is selected from the following compounds: N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide,
N-ε²⁶-(19-Carboxynonadecanoylamino)-4(S)-carboxybutanoyl-[Gly8, Arg34]GLP-1-(7-37) peptide,
N-ε²⁶-(19-Carboxynonadecanoylamino)-4(S)-carboxybutanoyl-[Arg34]GLP-1-(7-37) peptide,
N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide, or
N-ε²⁶-[2-(2-[2-(4-[21-Carboxynodecanoylamino]-4(S)-carboxybutanoylamino)ethoxy]ethoxy)acetyl][Gly8, Arg34]GLP-1-(7-37) peptide.

The inventor unexpectedly discovered that when the pharmaceutical combination or pharmaceutical composition of the present invention includes the GLP-1 receptor agonist shown in formula (B) of the present invention and PCSK9 inhibitor, it has better or equivalent hypoglycemic effects, longer or equivalent action time or half-life compared to the pharmaceuticalcomposition containing somalutide and PCSK9 inhibitor.

Optionally, the PCSK9 inhibitor is alirocumab or evolocumab.

Optionally, the content of the GLP-1 receptor agonist is about 0.05mg/ml-about 6.0mg/ml, 0.05mg/ml-about 5.0mg/ml, 0.05mg/ml-about 4.0mg/ml, 0.05mg/ml-about 3.0 mg/ml, 0.1 mg/ml-about 2.0 mg/ml, preferably about 0.1 mg/ml-about 1.0 mg/ml, preferably about 0.5 mg/ml-about 1.0 mg/ml;
the content of the PCSK9 inhibitor is about 5 mg/ml-about 420 mg/ml, preferably about 5 mg/ml-about 300 mg/ml, preferably about 5 mg/ml-about 200 mg/ml, preferably about 10 mg/ml-about 150mg/ml, preferably about 35mg/ml-140mg/ml, preferably about 35mg/ml-about 70mg/ml;
the content of the buffer is about 0.05mM-about 100mM, preferably about 0.05mM-about 85mM, preferably about 0.05mM-about 82mM, preferably about 0.05mM-about 40mM, preferably about 0.1mM-about 30mM, preferably about 1mM-about 20mM, preferably about 5mM-about 15mM, preferably about 5mM -about 10mM;
the content of the stabilizer is about 0.1% w/v-about 15% w/v, preferably about 0.5% w/v-about 10% w/v, preferably about 2.5% w/v-about 10% w /v; and
the content of the surfactant is about 0.001% w/v-about 10% w/v, preferably about 0.01% w/v-about 1% w/v.

Optionally, the pH of the pharmaceutical composition is about 4.0-about 8.0, preferably about 4.5-about 7.5, preferably about 4.5-about 7.0, preferably about 4.5-about 6.5, preferably about 4.5-about 6.2, preferably about 5.6-about 6.2, preferably about 6.0-about 6.2, preferably about 4.5-about 5.5, preferably about 5.0-about 5.5.

The present invention also provides a pharmaceutical composition, which comprises:
about 0.5-5.0 mg/ml, preferably about 0.5-3.0 mg/ml, preferably about 0.5-2.0 mg/ml, preferably about 0.5-1.0 mg/ml of N-ε²⁶-[2-(2-[2-(2-[2-(2-[4-(21 -carboxynodecanoylamino)-4(S)-carboxybutanoylamino]ethoxy)ethoxy [Gly]acetylamino)ethoxy]ethoxy)acetyl][Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl][Gly8, Arg34] GLP-1-(7-37) peptide;
about 35 mg/ml-140 mg/ml, preferably about 35 mg/ml-70 mg/ml of alirocumab or evolocumab;
about 8mM-about 20mM, preferably about 8mM-about 10mM of sodium acetate or disodium hydrogen phosphate;
about 2.0% w/v-about 10% w/v, preferably about 2.5% w/v-about 5.0% w/v of sucrose, histidine or proline;
about 0.01% w/v-about 0.1% w/v of polysorbate 20 or polysorbate 80; and a pH of about 4.5-about 6.5, 4.5-about 6.2, preferably about 5.6-about 6.2, preferably about 6.0-about 6.2, 4.5-about 5.5, preferably about 5.0-about 5.5.

The present invention also provides a pharmaceutical composition, which comprises:
about 0.5-5.0 mg/ml, preferably about 0.5-3.0 mg/ml, preferably about 0.5-2.0 mg/ml of N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide;
about 35 mg/ml to 140 mg/ml, preferably about 35 mg/ml to 70 mg/ml of alirocumab or evolocumab;
about 50mM-70 mM of disodium hydrogen phosphate and/or about 10mM-30mM of citric acid monohydrate, preferably about 60-65mM of disodium hydrogen phosphate and/or about 15mM-20mM of citric acid monohydrate; preferably about 63mM of disodium hydrogen phosphate and/ or approximately 18mM of citric acid monohydrate;
about 2.0% w/v to about 10% w/v, preferably about 2.5% w/v to about 5.0% w/v of sucrose, histidine or proline;
about 0.01% w/v to about 0.1% w/v of polysorbate 20 or polysorbate 80; and
a pH of about 4.5 to about 7.0, preferably about 4.5 to about 6.5, preferably 4.5 to about 6.2, preferably about 5.6 to about 6.2, preferably about 6.0 to about 6.2.

The third aspect of the present invention discloses a freeze-dried pharmaceutical compositionprepared through pharmaceutical combinations or pharmaceutical compositions disclosed in the first and/or second aspects.

The fourth aspect of the present invention discloses a kit, comprising:
(1) first therapeutic agent: GLP-1 receptor agonist defined in first aspect above;
(2) second therapeutic agent: it contains the PCSK9 inhibitor defined in first aspect above.

Optionally, the GLP-1 receptor agonist is selected from the following compounds: semaglutide, N-ε²⁶-(17-Carboxyheptadecanoylamino)-4(S)-carboxybutanoyl-[Arg34]GLP-1-(7-37) peptide, N-ε²⁶-(17-Carboxyheptadecanoylamino)-4(S)-carboxybutanoyl-[Gly8,Arg34]GLP-1-(7-37) peptide, N-ε²⁶-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl][ Gly8,Arg34]GLP-1-(7-37) peptide, N-ε³⁰-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val 8 Glu 22 Lys 30 Arg 26,34 -GLP-1(7-37)) peptide, N-ε²³-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val⁸ Glu²² Lys²³ Arg^{26,34}-GLP-1(7-37)) peptide, N-ε²⁶-[2-(2-[2-(2-[2-(2-[4-(19-Carboxynonadenylamino)-4(S)-carboxybutanoylamino]ethoxy)ethoxy]acetylamino)ethoxy]ethoxy)acetyl ][Gly8, Arg34]GLP-1-(7-37) peptide, N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide, N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide, N-ε²⁶-[2-(2-[2-(4-[21-Carboxynodecanoylamino]-4(S)-carboxybutanoylamino)ethoxy]ethoxy)acetyl][Gly8, Arg34]GLP-1-(7-37) peptide, N-ε²⁶-[2-(2-[2-(2-[2-(2-[4-(23-Carboxytricanoylamino)-4(S)-carboxybutanoylamino]ethoxy)ethoxy]acetylamino)ethoxy]ethoxy)acetyl ][Gly8, Arg34]GLP-1-(7-37) peptide, N-ε²⁶-[2-(2-[2-(4-[23-Carboxytricanoylamino]-4(S)-carboxybutanoylamino)ethoxy]ethoxy)acetyl][Gly8, Arg34]GLP-1-(7-37) peptide, N-ε²⁶-(23-Carboxytricosylamino)-4(S)-carboxybutanoyl-[Gly8, Arg34]GLP-1-(7-37) peptide, N-ε²⁶-(19-Carboxynonadecanoylamino)-4(S)-carboxybutanoyl-[Gly8, Arg34]GLP-1-(7-37) peptide, N-ε²⁶-(21-Carboxynodecanoylamino)-4(S)-carboxybutanoyl-[Gly8, Arg34]GLP-1-(7-37) peptide, N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Arg34]GLP-1-(7-37) peptide, N-ε²⁶-[2-(2-[2-(4-[19-Carboxynonadecanoylamino]-4(S)-carboxybutanoylamino)ethoxy]ethoxy)acetyl][Arg34]GLP-1-(7-37)peptide, N-ε²⁶-[2-(2-[2-(2-[2-(2-[4-(21-carboxynodecanoylamino)-4(S)-carboxybutanoylamino]ethoxy)ethoxy [Arg34]GLP-1-(7-37) peptide, N-ε²⁶-[2-(2-[2-(4-[21-Carboxynodecanoylamino]-4(S)-carboxybutanoylamino)ethoxy]ethoxy)acetyl][Arg34]GLP-1-(7-37)peptide, N-ε²⁶-[2-(2-[2-(2-[2-(2-[4-(23-Carboxytricanoylamino)-4(S)-carboxybutanoylamino]ethoxy)ethoxy [Arg34]GLP-1-(7-37) peptide, N-ε²⁶-[2-(2-[2-(4-[23-Carboxytricosanoylamino]-4(S)-carboxybutanoylamino)ethoxy]ethoxy)acetyl][Arg34]GLP-1-(7-37)peptide, N-ε²⁶-(23-Carboxytricosanoylamino)-4(S)-carboxybutanoyl-[Arg34]GLP-1-(7-37) peptide, N-ε²⁶-(19-Carboxynonadecanoylamino)-4(S)-carboxybutanoyl-[Arg34]GLP-1-(7-37) peptide, N-ε²⁶-(21-Carboxynodecanoylamino)-4(S)-carboxybutanoyl-[Arg34]GLP-1-(7-37) peptide, N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Gly8, Arg34]GLP-1-(7-37) peptide, N-ε²⁶-[2-(2-[2-(4-[20-Carboxyicosanoylamino]-4(S)-carboxybutanoylamino)ethoxy]ethoxy)acetyl][Gly8, Arg34]GLP-1-(7-37) peptide, N-ε²⁶-[2-(2-[2-(2-[2-(2-[4-(22-Carboxydodecanoylamino)-4(S)-carboxybutanoylamino]ethoxy)ethoxy]acetylamino)ethoxy]ethoxy)acetyl ][Gly8, Arg34]GLP-1-(7-37) peptide, N-ε²⁶-[2-(2-[2-(4-[22-Carboxydodecanoylamino]-4(S)-carboxybutanoylamino)ethoxy]ethoxy)acetyl][Gly8, Arg34]GLP-1-(7-37) peptide, N-ε²⁶-(20-Carboxyeicosanoylamino)-4(S)-carboxybutanoyl-[Gly8, Arg34]GLP-1-(7-37) peptide, N-ε²⁶-(22-Carboxydodecanoylamino)-4(S)-carboxybutanoyl-[Gly8, Arg34]GLP-1-(7-37) peptide, N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Arg34]GLP-1-(7-37) peptide, N-ε²⁶-[2-(2-[2-(4-[20-Carboxyeicosanoylamino]-4(S)-carboxybutanoylamino)ethoxy]ethoxy)acetyl][Arg34]GLP-1-(7-37)peptide, N-ε²⁶-[2-(2-[2-(2-[2-(2-[4-(22-Carboxydodecanoylamino)-4(S)-carboxybutanoylamino]ethoxy)ethoxy [Arg34]GLP-1-(7-37) peptide, N-ε²⁶-[2-(2-[2-(4-[22-Carboxydodecanoylamino]-4(S)-carboxybutanoylamino)ethoxy]ethoxy)acetyl][Arg34]GLP-1-(7-37)peptide, N-ε²⁶-(20-Carboxyeicosanoylamino)-4(S)-carboxybutanoyl-[Arg34]GLP-1-(7-37) peptide, or N-ε²⁶-(22-Carboxydocosanoylamino)-4(S)-carboxybutanoyl-[Arg34]GLP-1-(7-37) peptide;
Preferably, the compound is selected from the following compounds: N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Gly8, Arg34]GLP-1-(7-37) peptide, N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide, N-ε²⁶-(19-Carboxynonadecanoylamino)-4(S)-carboxybutanoyl-[Gly8, Arg34]GLP-1-(7-37) peptide, N-ε²⁶-(19-Carboxynonadecanoylamino)-4(S)-carboxybutanoyl-[Arg34]GLP-1-(7-37) peptide, N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide, or N-ε²⁶-[2-(2-[2-(4-[21-Carboxynodecanoylamino]-4(S)-carboxybutanoylamino)ethoxy]ethoxy)acetyl][Gly8, Arg34]GLP-1-(7-37) peptide.

Optionally, PCSK9 inhibitor is alirocumab or evolocumab.

Optionally, the first therapeutic agent is a freeze-dried powder product. Optionally, the second therapeutic agent further comprises:
buffer, the buffer is selected from at least one from the group consisting of citric acid monohydrate, citrate, sodium acetate, sodium dihydrogen phosphate, glutamate, disodium hydrogen phosphate, and trishydroxymethylaminomethane buffer , preferably the buffer is selected from at least one from the group consisting of citric acid monohydrate, sodium acetate and disodium hydrogen phosphate;
stabilizer, the stabilizer is selected from at least one from the group consisting of polyhydroxy hydrocarbons, disaccharides, benzyl alcohol, amino acids, polyols, and phenol, preferably, the stabilizer is selected from at least one from the group consisting of sucrose, lactose, fructose, maltose, trehalose, sorbitol,, mannitol, arginine, lysine, methionine, taurine, histidine and proline; preferably the stabilizer is selected from at least one from the group consisting of histidine, proline and sucrose.; and
surfactant, the surfactant is selected from at least one from the group consisting of polysorbate 80, polysorbate 20, and poloxamer 188.

Optionally, the kit comprises:
(1) first therapeutic agent: comprising about 0.05 mg to about 6.0 mg, 0.05 mg to about 5.0 mg, 0.05 mg to about 4.0 mg, 0.05 mg to about 3.0 mg, 0.1 mg to about 2.0 mg, preferably about 0.1 mg to about 1.0 mg, preferably about 0.5 mg to about 1.0 mg of GLP-1 receptor agonist;
(2) second therapeutic agent: comprising about 5 mg/ml to about 420 mg/ml, preferably about 5 mg/ml to about 300 mg/ml, preferably about 5 mg/ml to about 200 mg/ml, preferably about 10 mg /ml-about 150 mg/ml, preferably about 35 mg/ml-140 mg/ml, preferably about 35 mg/ml-about 70 mg/ml of the PCSK9 inhibitor;

about 0.05mM to about 100mM, 0.05mM to about 85mM, preferably about 0.05mM to about 82mM, 0.05mM to about 40mM, preferably about 0.1mM to about 30mM, preferably about 1mM to about 20mM, preferably about 5mM to about 15mM, preferably about 5mM to about 10mM of buffer;
about 0.1% w/v to about 15% w/v, preferably about 0.5% w/v to about 10% w/v, preferably about 2.5% w/v to about 10% w/v of stabilizer;
about 0.001% w/v to about 10% w/v, preferably about 0.01% w/v to about 1% w/v of surfactant; and
about 4.0 to about 8.0, preferably about 4.5 to about 7.5, preferably about 4.5 to about 7.0, preferably about 4.5 to about 6.5, preferably about 5.6 to about 6.2, preferably about 4.5 to about 6.2, further preferably about 4.5 to about 6.0 of pH.

Optionally, the kit comprises:
(1) first therapeutic agent: about 0.5 mg to about 5.0 mg, about 0.5 mg to about 3.0 mg, about 0.5 mg to about 2.0 mg, preferably about 0.5 mg to about 1.0 mg of N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide;
(2) second therapeutic agent: about 35 mg/ml to 140 mg/ml, preferably about 35 mg/ml to 70 mg/ml of alirocumab or evolocumab;

about 8mM to about 20mM, preferably about 8mM to about 10mM of sodium acetate or disodium hydrogen phosphate;
about 2.0% w/v to about 10% w/v, preferably about 2.5% w/v to about 5.0% w/v of sucrose, histidine or proline;
about 0.01% w/v to about 0.1% w/v of polysorbate 20 or polysorbate 80; and
about 4.5 to about 6.5, 4.5 to about 6.2, 4.5 to about 6.0, preferably about 5.0 to about 5.5 of pH.

Optionally, the kit comprises:
(1) first therapeutic agent: about 0.5 mg to about 5.0 mg, about 0.5 mg to about 3.0 mg, about 0.5 mg to about 2.0 mg, or preferably about 0.5 mg to about 1.0 mg of N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide;
(2) second therapeutic agent: about 35 mg/ml-140 mg/ml, preferably about 35 mg/ml-70 mg/ml of alirocumab or evolocumab;

about 50mM-70Mm disodium of hydrogen phosphate and/or about 10mM-30mM of citric acid monohydrate, preferably about 60-65mM of disodium hydrogen phosphate and/or about 15mM-20mM of citric acid monohydrate; preferably about 63mM of disodium hydrogen phosphate and /or approximately 18mM of citric acid monohydrate;
about 2.0% w/v to about 10% w/v, preferably about 2.5% w/v to about 5.0% w/v of sucrose, histidine or proline;
about 0.01% w/v to about 0.1% w/v of polysorbate 20 or polysorbate 80; and
about 4.5 to about 7.0, preferably about 4.5 to about 6.5, preferably about 4.5 to about 6.2, preferably about 4.5 to about 6.0, preferably about 5.6 to about 6.2, preferably about 5.0 to about 5.5 of pH.

The fifth aspect of the invention discloses the use of the pharmaceutical combination in the first aspect, the pharmaceutical composition in the second aspect, the freeze-dried pharmaceutical composition in the third aspect, or the kit in the fourth aspect in the preparation of a medicament for treating hyperglycemia, diabetes, obesity and/or cholesterol related symptoms.

The invention also discloses a pharmaceutical combination in the first aspect, a pharmaceutical composition in the second aspect, a freeze-dried pharmaceutical composition in the third aspect, or a kit in the fourth aspect,for use in the treatment of hyperglycemia, diabetes, obesity, and/or cholesterol related symptoms.

The invention also discloses a method for treating hyperglycemia, diabetes, obesity and/or cholesterol related symptoms, comprising administrating a therapeutically effective amount of the pharmaceutical combination in the first aspect, the pharmaceutical composition in the second aspect, the freeze-dried pharmaceutical composition in the third aspect, or a kit in the fourth aspect.

### Definitions:

Certain terms used herein are described below. It is understood that, unless otherwise defined, terms used herein have the same meaning as commonly understood by those skilled in the art to which the present invention belongs. And, the term used herein for the purpose of describing particular examples solely and it is not intended to be used as a limitation, as the scope of the invention is limited only by the appended claims.

In this application, the terms "about" or "approximately" as used herein generally mean within 20%, preferably within 10%, more preferably within 5% of a given value or range when referring to specifically enumerated values or ranges of values.

In this application,the term "w/v" herein refer to a unit of concentration representing grams per milliliter (g/ml).

In this application, the term " pharmaceutical combination" herein refers to a fixed combination, or a non-fixed combination, or a kit in which two or more therapeutic agents may be administered simultaneously or separately, in the form of a single dosage unit.

In this application, the term "fixed combination" or "fixed dose" herein refers to a single dosage form formulated to deliver a specific amount of two therapeutic agents to a patient, with the combined quantity being effective for the treatment of a disease. The single dosage form is designed to deliver an amount of each agent together with any pharmaceutically acceptable carriers or excipients, i.e., that is, the two agents in one dosage form, are administered simultaneously to the patient to be treated together.

In this application, the term "non-fixed combination" herein refers to the active ingredients, such as a GLP-1 receptor agonist and a PCSK9 inhibitor, are administered simultaneously, concurrently, or sequentially to the patient as separate entities, with no specific time limitations.

In this application, the term "kit" herein refers to a set of products or a package of combination products, i.e., the first therapeutic agent GLP-1 receptor agonist and the second therapeutic agent PCSK9 inhibitor of the present invention are sold in the same package. For example, a specific example comprises a container containing a therapeutic agent (said container comprising, e.g., a bottle, vials, syringes, injection pens, foam packages,etc.), while it also contain instructions on the simultaneous, sequential, separate, or mixed administration of the two therapeutic agents, such as labels or directions,, and it may also contain other substances that is desirable from commercial and user perspective, including diluents, needles, syringes, etc. In other example, the kit comprises a first container with a first therapeutic agent, a GLP-1 receptor agonist therein, and a second container containing a second therapeutic agent, a PCSK9 inhibitor therein; and in another example, the kit comprises a two-chambered or multi-chambered container, with the first therapeutic agent, a GLP-1 receptor agonist therein, and the second therapeutic agent, a PCSK9 inhibitor therein, housed separately in different chambers.

In this application, the term "pharmaceutical composition" or "formulation" herein comprises at least one active ingredient, such as a) a GLP-1 receptor agonist, and/or b) a PCSK9 inhibitor, and a pharmaceutically usable carrier, diluent, or excipient.

In this application, the term "excipient" herein refers to any non-therapeutic substance added to a formulation to provide a desired consistency, viscosity, or stability, etc.

In this application, the term "antibody and antigen-binding portion thereof" is used herein in its broadest sense, and includes a wide variety of antibody structures including, but not limited to monoclonal antibodies, polyclonal antibodies, multi-specific antibodies (e.g., bispecific antibodies), and antibody fragments, as long as they exhibit the desired antigen-binding activity.

In this application, the term "antibody" generally refers to an immunoglobulin molecule comprising four polypeptide chains, i.e., two heavy (H) chains and two light (L) chains, interconnected by disulfide bonds, and a multimer thereof (e.g., IgM). However, immunoglobulin molecules comprising only heavy chains (i.e., lacking light chains) are also included in the definition of the term "antibody". Each heavy chain contains heavy chain variable region (abbreviated herein as HCVR or VH) and heavy chain constant region. The heavy chain constant region contains three structural domains: CH1, CH2 and CH3. Each light chain contains a light chain variable region (abbreviated herein as LCVR or VL) and a light chain constant region. The light chain constant region contains one structural domain (CL1). The VH and VL regions can be further subdivided into highly variable regions called complementary decision region (CDR), which alternate with more conserved regions called framework region (FR). Each VH and VL contains three CDRs and four FRs, arranged in the following order from the amino-terminal to the carboxy-terminal: FR1, CDR1, FR2, CDR2, FR3, CDR3, FR4.

As used herein, the term "antigen-binding portion" or "antigen-binding fragment" ("antibody portion" or "antibody fragment") of an antibody refers to one or more fragments of the antibody that retain the ability to bind specifically to PCSK9. The term "antigen-binding portion" (or "antibody portion" or "antibody fragment") of an antibody herein refers to one or more fragments of an antibody that retains the ability to bind specifically to an antigen (e.g., RSV-F protein). It has been shown that the antigen-binding function of an antibody can be achieved by certain fragments of a full-length antibody. The term "antigen-binding portion" of an antibody encompasses binding fragments that include (i) Fab fragments, i.e., monovalent fragments consisting of the VL, VH, CL1, and CH1 domains; (ii) F(ab')₂ fragments, i.e., bipartite fragments consisting of two F(ab') fragments connected by a disulfide bond in the hinge region; and (iii) the Fd fragment consisting of the VH and CH1 structural domains; (iv) the Fv fragment consisting of the VL and VH structural domains of the single arm of the antibody; (v) the dAb fragment consisting of the VH structural domains; and (vi) the CDR. In addition, although the two structural domains of the Fv fragment, VL and VH, are encoded by different genes, they can be linked together by recombination by a synthetic connector to form separate linked chains in which the VL and VH regions are paired to form a monovalent molecule (referred to as single-chain Fv (scFv)). Such single-chain antibodies are also covered by the term "antigen-binding portion" of the antibody. Other forms of single-chain antibodies, such as bispecific antibodies, are also covered.

In this application, the term "specific binding" herein refers to the formation of a complex between an antibody or antigen-binding fragment thereof and an antigen that is relatively stable under physiological conditions. Specific binding can be characterized by a dissociation constant of at least about 1×10⁻⁶M or greater. Methods for determining whether two molecules are specifically bound are well known in the art and include, for example, equilibrium dialysis, surface equilibrium kinetics, etc.

### GLP-1 analogs, and GLP-1 derivatives

As used herein, the term "GLP-1 analog" or "analog of GLP-1" refers to a peptide or compound that is a variant of human glucagon-like peptide-1 (GLP-1(7-37)), wherein one or more amino acid residues of GLP-1(7-37) are replaced, and/or wherein one or more amino acid residues are deleted, and/or wherein one or more amino acid residues are added. Specifically, the sequence of GLP-1(7-37) is as shown in SEQ ID NO: 1 in the Sequence Table. Peptides having the sequence shown in SEQ ID NO: 1 may also be referred to as "natural" GLP-1 or "natural" GLP-1(7-37).

In the sequence table, the first amino acid residue (histidine) of SEQ ID NO: 1 is numbered 1. Hereinafter, in accordance with established practice in the art, this histidine residue is numbered as 7 and subsequent amino acid residues are numbered accordingly, ending with Gly 37. Thus, in general, the amino acid residue numbering or position numbering of the GLP-1 (7-37) sequences covered herein is that of a sequence beginning with His at position 7 and ending with Gly at position 37.

The [Gly8, Arg34] GLP-1-(7-37) peptide, a GLP-1 analog having Gly and Arg at positions corresponding to position 8 and position 34, respectively, of GLP-1(7-37) (SEQ ID NO: 1). The [Arg34] GLP-1-(7-37) peptide, a GLP-1 analog having Gly and Arg at positions corresponding to position 34, of GLP-1(7-37) (SEQ ID NO: 1). Specifically, the amino acid sequences of the [Gly8, Arg34]GLP-1-(7-37) peptide and the [Arg34]GLP-1-(7-37) peptide are shown as SEQ ID NO: 2 and SEQ ID NO: 3 in the Sequence Table, respectively.

In the case of a GLP-1 peptide or analog thereof, the term "derivative" herein refers to a chemically modified GLP-1 peptide or analog thereof in which one or more substituents have been covalently linked to said peptide. The substituents may also be referred to as side chains.

Unless otherwise indicated, reference to acylation with a lysine residue is understood to be carried out with its ε-amino group.

The GLP-1 derivatives of formula (B) of the present invention may exist in different stereoisomeric forms having the same molecular formula and attached atoms sequence, but differing only in the three-dimensional orientation of their atomic space. Unless otherwise indicated, the present invention relates to all stereoisomeric forms of the derivatives that are subject to protection.

The term "peptide" when used, for example, with respect to the GLP-1 analogs of the present invention, refers to a compound comprising a series of amino acids interconnected by amide (or peptide) bonds.

In one specific example, the peptide consists largely or predominantly of amino acids interconnected by amide bonds (e.g., at least 50%, 60%, 70%, 80%, or at least 90% by molar mass). In another specific example, the peptide comprises amino acids interconnected to each other by peptide bonds.

Amino acids are molecules containing amino and carboxylic acid groups, optionally containing one or more additional groups, commonly referred to as side chains.

The term "amino acid" encompasses proteinaceous amino acids (encoded by the genetic code, including natural and standard amino acids), as well as non-proteinaceous (not found in proteins and/or not encoded by the standard genetic code), and synthetic amino acids. Non-proteinogenic amino acids are portions that can be incorporated into a peptide by peptide bonding, but are not proteinogenic amino acids. Synthetic non-proteinogenic amino acids include amino acids produced by chemical synthesis, i.e., D-isomers of amino acids encoded by the genetic code such as D-alanine and D-leucine, Aib (α-aminoisobutyric acid), Abu (α-aminobutyric acid), 3-aminomethylbenzoic acid, o-aminobenzoic acid, deamino-histidine, β-analogues of amino acids such as β-alanine, etc., D-histidine, deamino-histidine, 2-amino-histidine, β-hydroxy-histidine, and homohistidine, etc.

Non-limiting examples of amino acids not encoded by the genetic code are γ-carboxyglutamic acid, ornithine, D-alanine, D-glutamine and phosphoserine. Non-limiting examples of synthetic amino acids are the D-isomers of amino acids, such as D-alanine and D-leucine, Aib (α-aminoisobutyric acid), β-alanine, and des-amino-histidine (desH, alternatively known as imidazolepropionic acid, abbreviated Imp).

All following amino acids that are not labeled as spin isomers are understood to refer to the L-isomer (unless otherwise indicated).

Methods of preparing peptides and GLP-1 analogs of GLP-1 (7-37) of the present invention are well known in the art. For example, the GLP-1 peptide portions (or fragments thereof) of the derivatives of the present invention, as well as the GLP-1 analogs of the present invention, can be produced by classical peptide synthesis, such as solid-phase peptide synthesis using t-Boc or Fmoc chemistry, or by other well-established techniques, see, e.g. Greene and Wuts, "Protective Groups in Organic Synthesis" , John Wiley &Sons,1999, Florencio Zaragoza "Organic Synthesis on solid Phase" , Wiley-VCH Verlag GmbH, 2000, and edited by W.C.Chan and P.D.White, "Fmoc Solid PhasePeptide Synthesis", Oxford University Press, 2000.

In one example, the intact GLP-1 analogs of the present invention such as [Gly8, Arg34]GLP-1-(7-37) peptides can be generated by recombinant methods, i.e., by culturing a host cell that contains a DNA sequence encoding the analog and is capable of expressing the peptide in a suitable nutrient medium under conditions permitting expression of the peptide. Non-limiting examples of host cells suitable for expression of these peptides are Escherichia coli, Saccharomyce scerevisiae and mammalian BHK or CHO cell lines. In some examples, this fully recombinant fermentation step of the production process method is satisfying, e.g., for production economics considerations.

The fusion protein inclusion bodies containing the main chain of the GPL-1 compound are denatured and renatured to obtain fusion proteins containing the correct conformation, which are subjected to a series of treatments such as enzymatic cleavage, tonic precipitation, and centrifugation to obtain a higher content of the main chain of the GLP-1 compound. Purified by ion exchange chromatography, the main chain of GLP-1 compound with higher purity was obtained after treatment.

The term "amino acid residue" encompasses an amino acid from which the hydrogen atom has been removed from the amino group and/or the hydroxyl group has been removed from the carboxyl group and/or the hydrogen atom has been removed from the sulfhydryl group. Imprecisely, an amino acid residue may be called an amino acid.

Unless otherwise indicated, amino acids referred to herein are L-amino acids.

The term "alkylene glycol" includes oligo- and polyalkylene glycol portions, as well as monomeric alkylene glycol portions. Monomeric alkylene glycol and polyalkylene glycol include chains based on mono- and polyethylene glycol, mono- and polypropylene glycol, and mono- and polybutylene glycol, i.e., chains based on repeating units -CH2CH2O-, - CH2CH2CH2O-, or -CH2CH2CH2CH2O-. The alkylene glycol portion can be monodisperse (having well-defined length/molecular weight) as well as polydisperse (having less well-defined length/average molecular weight). Monomeric alkylene glycol portions include chains with different end groups, such as -OCH2CH2O- , -OCH2CH2CH2O- or - OCH2CH2CH2CH2O-.

The term "fatty acid" includes straight or branched chain aliphatic carboxylic acids having at least two carbon atoms and being saturated or unsaturated. Non-limiting examples of fatty acids are, for example, myristic acid, palmitic acid, stearic acid and eicosanoic acid.

Herein, the term " fatty diacid " includes straight or branched chain aliphatic dicarboxylic acids having at least two carbon atoms and saturated or unsaturated. Non-limiting examples of fatty diacid are adipic acid, octanedioic acid, decanedioic acid, dodecanedioic acid, tetradecanedioic acid, hexadecanedioic acid, heptadecanedioic acid, octadecanedioic acid, eicosanedioic acid, docosanedioic acid, and tetracosanedioic acid.

In this invention, GLP-1 compounds are named according to the following principles: names are given by mutation and modification (e.g., acylation) relative to the natural GLP-1 (7-37). For the naming of the acyl portion, the naming is done according to IUPAC nomenclature and in other cases according to peptide nomenclature. For example, the acyl portion is named as described below:

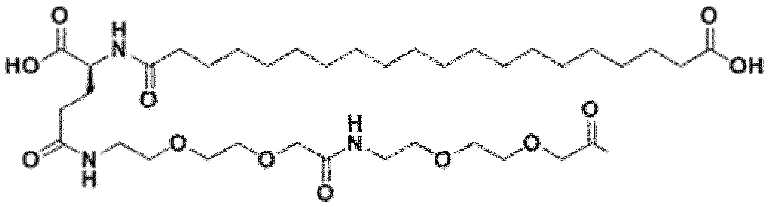

It can be named, for example, as "eicosanedioyl-yGlu-OEG-OEG", "eicosanedioyl-γGlu-2×OEG" or "eicosanedioyl-gGlu-2×OEG", "19-carboxynonadecanoyl-γGlu-2×OEG" or "19-carboxynonadecanoyl-yGlu-OEG-OEG", wherein OEG is the shorthand for the group - NH(CH 2 ) 2 O(CH 2 ) 2 OCH 2 CO- (i.e., 2-[2-(2-aminoethoxy)ethoxy]acetyl) and yGlu (or gGlu) is the shorthand for the amino acid γ-glutamic acid in the L configuration. Alternatively, the acyl moieties may be named according to IUPAC nomenclature (OpenEye, IUPAC format). According to this nomenclature, the above acyl moiety of the present invention is referred to as the following name: "[2-[2-[2-[2-[2-[2-[(4S)-4-carboxy-4-(19-carboxynonadecanoylamino)butanoyl] -amino -ethoxy] - ethoxy]acetyl]amino]ethoxy]ethoxy]acetyl]" or "[2-(2-[2-(2-[2-(2-[4-(19-carboxynonadecanoylamino)-4(S)-carboxybutanoylamino] ethoxy)ethoxy] acetylamino)ethoxy]ethoxy)acetyl ]" .

In the present application, the term "synergistic effect" can be determined by the Zheng-Jun Jin's Q value, wherein the Q value is obtained by the following formula: Q=E_{a+b}/(Eₐ+E_{b}-Eₐ×E_{b}); wherein E_{a+b} denotes the decline rate of detection index of the combination or mixed administration of two drugs, and Eₐ, E_{b} denote the decline rate of respective detection indexs of the two drugs used alone. If Q is between 0.85 and 1.15, it means that the two drugs are additive; Q>1.15, they are synergistic; Q<0.85, they are antagonistic.

"Dialysis replacement buffer" is the use of semi-permeable membrane to wrap the protein solution, immersed in the dialysis buffer, because the concentration of the buffer is lower than the concentration of the membrane, therefore the exchange of substances will occur on the semi-permeable membrane; however, proteins cannot pass through the semi-permeable membrane, only small molecules, such as salt ions, can pass through.

The parmaceutical combination/parmaceutical composition provided by the present invention has the following advantages:
1) The parmaceutical combinations/parmaceutical compositions/lyophilized formulations described in the present invention maintain the effect of PCSK9 inhibitors in lipid-lowering action and maintain GLP-1 function or unexpectedly demonstrate synergistic effects in blood glucose-lowering action.
2) The pharmaceutical combinations/ pharmaceutical compositions/lyophilized formulationsherein bridge a gap in the field of treatment of patients suffering from both hyperglycemia and cholesterol-related symptoms.
3) The pharmaceutical combination/ pharmaceutical composition/lyophilized formulation herein has no significant difference in pharmacokinetic properties from the single drug and has good pharmacokinetic properties.
4) The parmaceutical combination/ parmaceutical composition/lyophilized formulation herein has good stability, and the components therein satisfy the internal control quality standards after being placed for several months.

### Brief Description of the Figures

Figure 1 shows the results of weight of humanized mice, horizontal axis: time (days), vertical axis: body weight (g), K0-K5 are the experimental groups;
Figure 2 shows the results of the change rate of body weight in humanized mice, horizontal axis: time (days), vertical axis: change rate of body weight (%), K0-K5 are the experimental groups;
Figure 3 shows the results of fasting blood glucose in humanized mice, horizontal axis: time (days), vertical axis: fasting blood glucose (mmol) in mice, K0-K5 are the experimental groups;
Figure 4 shows the results of change rate of fasting blood glucose in humanized mice, horizontal axis: time (days), vertical axis: change rate of fasting blood glucose in mice (%), K0-K5 are the experimental groups;
Figure 5 shows the results of LDL-C in humanized mice, horizontal axis: time (days), vertical axis: LDL-C (mmol) in mice, K0-K5 are the experimental groups;
Figure 6a shows serum free PCSK9 results, horizontal axis: time (days), vertical axis: mouse serum PCSK9 (ng/mL) (vertical axis scale is 1000 per grid), K0-K5 are experimental groups;
Figure 6b shows serum free PCSK9 results, horizontal axis: time (days), vertical axis: mouse serum PCSK9 (ng/mL) (vertical axis scale is exponentially labeled), K0-K5 are experimental groups;
Figure 7 is the PK (pharmacokinetic) curves: horizontal axis: time (hours, h), vertical axis: serum antibody concentration (ng/mL), M0, M1 and M2 indicate each experimental group;
Figure 8 is the pharmacodynamic results, specifically, serum LDL-C levels in cynomolgus monkeys: horizontal axis: time (hours, h), vertical axis: LDL-C concentration (mmol/L), M0, M1 and M2 denote each experimental group;
Figure 9 is the pharmacodynamic results, specifically, serum PCSK9 levels in cynomolgus monkeys, horizontal axis: time (hours, h), vertical axis: serum PCSK9 concentration (ng/ml), M0, M1 and M2 denote each experimental group;
Figure 10: Compound 5 insoluble particles hypoglycemic effect test, horizontal axis: time (days), vertical axis: blood glucose (relative concentration).

### Detailed Description of the invention

Examples of the invention will be described in detail below in connection with Examples. The following Examples are intended to illustrate the invention solely and should not be considered as limiting the scope of the invention. Where the specific conditions are not indicated in the examples, conventional conditions or conditions recommended by the manufacturer were followed. The reagents or instruments used, whose manufacturer is not indicated, are conventional products available commercially.

### Abbreviations

Na₂HPO₄ is disodium hydrogen phosphate;
NaOH is sodium hydroxide;
OEG is amino acid residue -NH(CH₂)₂O(CH₂)₂OCH₂CO-;
OSu is succinimidyl-1-yloxy-2,5-dioxo-pyrrolidin-1-yloxy;
OtBu is oxy-tert-butyl;
HCl is hydrogen chloride;
γ Glu or gGlu is yL-glutamyl;
NHS is N-hydroxysuccinimide;
DCC is dicyclohexylcarbodiimide;
AEEA is 2-(2-(2-aminoethoxy)ethoxy)acetic acid;
OH is hydroxyl;
Gly is glycine;
Arg is arginine;
TFA is trifluoroacetic acid;
SEC-HPLC is Size Exclusion Chromatography-High Performance Liquid Chromatography;
CEX-HPLC is Cation Exchange Chromatography-High Performance Liquid Chromatography;
CE-SDS is capillary electrophoresis sodium dodecyl sulfate;
RPC is Reversed-Phase Chromatography.

### Preparation of GLP-1 receptor agonists

### Example 1

Title compound : N-ε²⁶-[2-(2-(2-(2-(2-(2-(2-(2-(4-(19-carboxynonadecanoylamino )-4(S)-carboxybutanoylamino)ethoxy)ethoxy]acetylamino)ethoxy]ethoxy]ethox y)acetyl] [Gly8, Arg34]GLP-1-(7-37) peptide (Compound 1)

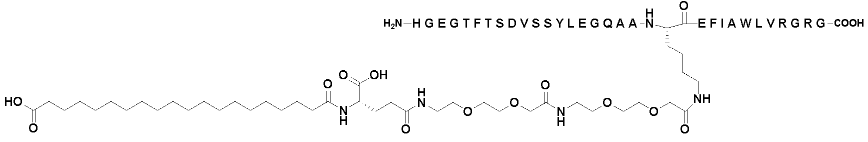

### 1. Preparation of N-ε²⁶-[2-(2-[2-(2-[2-(2-[4-(19-carboxynonadecanoylamino)-4(S)-carboxybutanoylamino]ethoxy)ethoxy]acetylamino)ethoxy]ethoxy)acetyl ][Gly8, Arg34]GLP-1-(7-37) peptide

[Gly8, Arg34]GLP-1-(7-37) peptide was prepared by a general protein recombinant expression method (for details, see Molecular Cloning: A Laboratory Manual (Fourth Edition), Michael R. Green, Cold Spring Harbor Press, 2012). [Gly8, Arg34]GLP-1-(7-37) peptide (5 g, 1.48 mmol) was dissolved in 100 mM aqueous Na₂HPO₄ solution (150 mL) and acetonitrile (100 mL) was added. The pH was adjusted to 10-12.5 with 1 N NaOH. Tert-butyl eicosanedioyl-γGlu(2×OEG-OSu)-OtBu (1.59 g, 1.63 mmol) was dissolved in acetonitrile (50 mL), and the solution was slowly added to a [Gly8, Arg34]GLP-1-(7-37) peptide solution. The pH value was maintained at 10-12.5. After 120 min, the reaction mixture was added to water (150 mL), and the pH value was adjusted to 5.0 with 1 N aqueous HCl. The precipitate was separated out by centrifugation and lyophilized. The crude product was added to a mixed solution of trifluoroacetic acid (60 mL) and dichloromethane (60 mL), and the mixture was stirred at room temperature for 30 min. The mixture was then concentrated to about 30 mL and poured into ice-cold n-heptane (300 mL), and the precipitated product was isolated by filtration and washed twice with n-heptane. The resulting precipitate was dried in vacuum and purified by ion exchange chromatography (Resource Q, 0.25%-1.25% ammonium acetate gradient in 42.5% ethanol, pH 7.5) and reverse phase chromatography (acetonitrile, water, TFA). The purified fractions were combined, adjusted to pH value 5.2 with 1 N HCl, and separated to obtain the precipitate, which was lyophilized to obtain the title compound.

LC-MS (ESI): m/z = 1028.79[M+4H]⁴⁺

### 2. Preparation of intermediate tert-butyl eicosanedioyl-γGlu-(2×OEG-OSu)-OtBu

### 2.1 Tert-butyl eicosanedioyl-OSu

Eicosanedioic acid mono-*tert*-butyl ester (20 g, 50.17 mmo1) and NHS (5.77 g, 50.17 mmo1) were mixed in dichloromethane (400mL) under nitrogen atmosphere, and triethylamine (13.95 mL) was added. The resulting turbid mixture was stirred at room temperature, added with DCC (11.39 g, 55.19 mmo1) and further stirred overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure and dried in vacuum overnight to obtain *tert*-butyl eicosanedioyl-OSu (24.12 g, yield 97%).

LC-MS (Scie×100API): m/z = 496.36(M+1)⁺

### 2.2 Tert-butyl eicosanedioyl-yGlu-OtBu

*Tert*-butyl eicosanedioyl-OSu (24.12 g, 48.66 mmol) was dissolved in dichloromethane (250 mL), and the solution was stirred and added with H-Glu-OtBu (10.88 g, 53.53 mmol), triethylamine (12.49 mL) and water (25 mL) sequentially. The mixture was heated to obtain a clarified solution, which was then stirred at room temperature for 4 h. Then, the reaction solution was added with 10% aqueous citric acid solution (200 mL) and subjected to liquid separation. The lower organic phase was washed with saturated brine, and after liquid separation, the lower organic phase was dried over anhydrous sodium sulfate and filtered, and the filtrate was concentrated to almost dryness under reduced pressure and dried in vacuum overnight to obtain *tert*-butyl eicosanedioyl-yGlu-OtBu (27.27 g, yield 96%).

LC-MS (Scie×100API): m/z = 584.44(M+1)⁺

### 2.3 Tert-butyl eicosanedioyl-yGlu-(OSu)-OtBu

*Tert*-butyl eicosanedioyl-yGlu-OtBu (27.27 g, 46.71 mmol) was dissolved in dichloromethane (300 mL) under nitrogen atmosphere, and triethylamine (11.99 mL) was added. The mixture was stirred for 10 min, and NHS (5.38 g, 50.17 mmol) was added, followed by the addition of DCC (10.60 g, 51.38 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure. *Tert*-butyl methyl ether was added, and the mixture was stirred for 30 min and filtered in vacuum. The filter cake was dried in vacuum overnight to obtain tert-butyl eicosanedioyl-yGlu-(OSu)-OtBu (25.76 g, yield 81%).

LC-MS (Scie×100API): m/z = 681.46(M+1)⁺

### 2.4 Tert-butyl eicosanedioyl-γGlu-(2×OEG-OH)-OtBu

*Tert*-butyl eicosanedioyl-yGlu-(OSu)-OtBu (25.76 g, 37.83 mmol) was dissolved in dichloromethane (250 mL), and the solution was stirred and added with 2× AEEA (11.66 g, 37.83 mmol), triethylamine (9.71 mL) and water (25 mL) sequentially. The mixture was heated to obtain a clarified solution, which was then stirred at room temperature for 4 h. Then, the reaction solution was added with 10% aqueous citric acid solution (200 mL) and subjected to liquid separation. The lower organic phase was washed with saturated brine, and after liquid separation, the lower organic phase was dried over anhydrous sodium sulfate and filtered, and the filtrate was concentrated to almost dryness under reduced pressure and dried in vacuum overnight to obtain *tert*-butyl eicosanedioyl-γGlu-(2×OEG-OH)-OtBu (30.75 g, yield 93%).

LC-MS (Scie×100API): m/z = 874.59(M+1)⁺

### 2.5 Tert-butyl eicosanedioyl-yGlu-(2×OEG-OSu)-OtBu

*Tert*-butyl eicosanedioyl-γGlu-(2×OEG-OH)-OtBu (30.75 g, 35.18 mmol) was dissolved in dichloromethane (300 mL) under nitrogen atmosphere, and triethylamine (9.03 mL) was added. The mixture was stirred for 10 min, and NHS (4.05 g, 35.18 mmol) was added, followed by the addition of DCC (7.98 g, 38.70 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure and dried in vacuum overnight to obtain *tert*-butyl eicosanedioyl-γGlu-(2×OEG-OSu)-OtBu (31.09 g, yield 91%).

LC-MS (Scie×100API): m/z = 971.61(M+1)⁺

### Example 2.

Title compound: *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide (Compound 2)

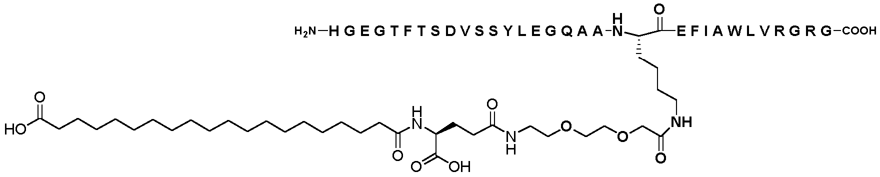

*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide was prepared by procedures similar to those described in section 1 of Example 1.

LC-MS (ESI): m/z = 992.52[M+4H]⁴⁺
The intermediate *tert*-butyl eicosanedioyl-γGlu-(OEG-OSu)-OtBu was prepared by procedures similar to those described in section 2 of Example 1.

LC-MS (Scie×100API): m/z = 826.54(M+1)⁺

### Example 3.

Title compound: *N*-ε²⁶-(19-carboxynonadecanoylamino)-4(*S*)-carboxybutanoyl-[Gly8, Arg34]GLP-1-(7-37) peptide (Compound 3)

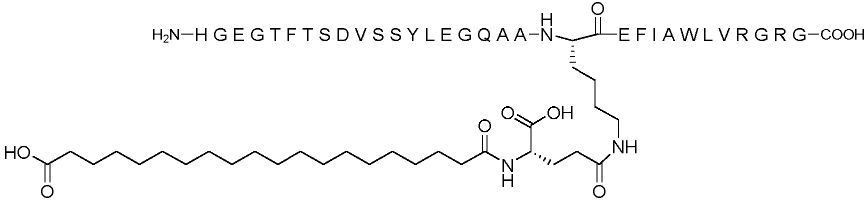

*N*-ε²⁶-(19-carboxynonadecanoylamino)-4(*S*)-carboxybutanoyl-[Gly8, Arg34]GLP-1-(7-37) peptide was prepared by procedures similar to those described in section 1 of Example 1.

LC-MS (ESI): m/z = 956.25[M+4H]⁴⁺

The intermediate *tert*-butyl eicosanedioyl-γGlu-(OSu)-OtBu was prepared by procedures similar to those described in section 2 of Example 1.

LC-MS (Scie×100API): m/z = 681.46(M+1)⁺

### Example 4.

Title compound: *N*-ε²⁶-(19-carboxynonadecanoylamino)-4(*S*)-carboxybutanoyl-[Arg34]GLP-1-(7-37) peptide (Compound 4)

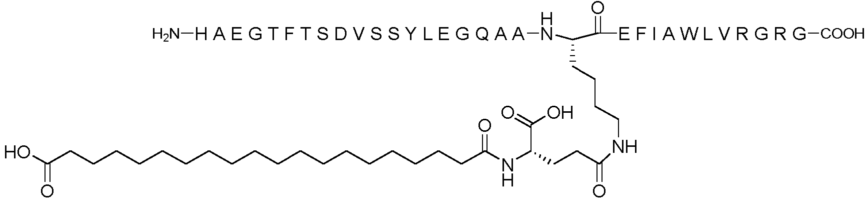

*N*-ε²⁶-(19-carboxynonadecanoylamino)-4(*S*)-carboxybutanoyl-[Arg34]GLP-1-(7-37)peptide was prepared by procedures similar to those described in section 1 of Example 1.

LC-MS (ESI): m/z = 959.75[M+4H]⁴⁺
The intermediate *tert-*butyl eicosanedioyl-γGlu-(OSu)-OtBu was prepared by procedures similar to those described in section 2 of Example 1. LC-MS (Scie×100API): m/z = 681.46(M+1)⁺

### Example 5

Title compound: N-ε²⁶-[2-(2-(2-(2-(2-(2-(2-(2-(4-(21-carboxyheneicosanoylamino)-4(S)-carboxybutanoylamino)ethoxy)ethoxy]acetylamino)ethoxy]ethoxy]ethox y)acetyl][Gly8, Arg34]GLP-1-(7-37) peptide (Compound 5)

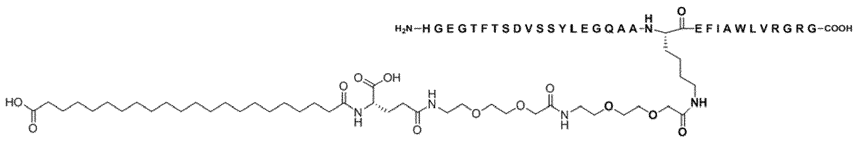

The N-ε²⁶-[2-(2-(2-(2-(2-(2-(2-(2-(4-(21-carboxyheneicosanoylamino)-4(S)-carboxybutanoylamino)ethoxy)ethoxy]acetylamino)ethoxy]ethoxy]ethox y)acetyl][Gly8, Arg34]GLP-1-(7-37) peptide was prepared by procedures similar to those described in section 1 of Example 1.

LC-MS (electrospray): m/z = 1035.80 [M+4H]⁴⁺
The intermediate tert-butyl docosanediyl-yGlu-(2xOEG-OSu)-OtBu was prepared in a similar procedure as in the section 2 of Example 1.

LC-MS (Scie x 100API): m/z = 999.64 (M+1)+

### Example 6

Semaglutide, prepared according to Example 4 of Patent CN101133082A.

### Source of PCSK9 Antibody

Alirocumab: The source of Alirocumab used in the present invention is Praluent^{®}, commercially available from Sanofi.

Evolocumab: The source of Evolocumab used in the present invention is Repatha^{®}, commercially available from Amgen.

### Animal experiments

Example 7 Effect of the combination of Alirocumab and semaglutide on blood glucose

### 1. Experimental method

The experimental animals in this Example were wild-type hamsters, and the hamsters were grouped and administered according to the following standard:
1) Control group: injection of excipient samples, subcutaneous injection (administration of 2 µL/kg), once every two days;
2) Alirocumab group: injection of Alirocumab sample (Alirocumab 10 mg/kg), subcutaneous injection, once every two days;
3) Semaglutide group: injection of semaglutide sample ( semaglutide 0.12mg/kg), subcutaneous injection, once every two days;
4) Alirocumab + semaglutide group: injection of Alirocumab (Alirocumab 10mg/kg) and semaglutide ( semaglutide 0.12mg/kg) by subcutaneous injection, the frequency of administration was once every two days.

Sample preparation for each group:
Excipient samples: the excipient contains 8 mM histidine, 10% sucrose (w/v) and 0.01% polysorbate 20 (w/v), which was adjusted to pH 6.0.
Alirocumab Sample: the excipient contains 8mM histidine, 10% sucrose (w/v) and 0.01% polysorbate 20 (w/v), pH=6. Preparation was performed by diluting commercially available Praluent^{®} (Alirocumab: 75mg/mL) with the above excipient into 5mg/ml, to obtain a 5mg/mL sample of the Alirocumab formulation.
Semaglutide Sample: the excipient contains 7.95 mM disodium hydrogen phosphate, 1.4% propylene glycol (w/v) and 0.55% phenol (w/v), pH=7.4. The preparation was to weigh semaglutide powder and add the above excipient to dissolve it into a 0.5 mg/ml semaglutide solution, which was then diluted with the above excipient to form a 0.06 mg/mL sample of semaglutide solution.
Alirocumab+ semaglutide parmaceutical combination samples: 5 mg/mL Alirocumab solution and 0.06 mg/mL semaglutide solution were prepared according to the preparation method of Alirocumab samples and semaglutide samples mentioned above, and the two solutions were injected into the experimental animals respectively.

On the first day of the experiment, each group of wild-type hamsters was given high-fat and high-sugar feed (containing 10% lard, 10% cholesterol, and 60% fructose) and injected with drugs according to the above dosing regimen for each group, and blood was collected from the inner canthus vein of the hamster on the day before administrating and 7^{th} day after dosing, plasma was anticoagulated with sodium heparin, and plasma was obtained by centrifugation of the whole blood at 4°C for 10 min at 4000 rpm, and the blood glucose level was detected by glucose assay kit (Biosino Bio-Technology and Science Incorporation.).

### 2. Data processing and experimental results

The obtained blood glucose data were normalized according to the following method: taking the blood glucose of each group before the administration of the drug as the baseline, the relative concentration of the corresponding time point obtained by comparing the blood glucose at the monitoring point after the administration of the drug to the baseline glucose, the relative concentration of blood glucose was expressed as x̅̅± s, and the decline rate of the blood glucose of each administration group was calculated by using the analysis of variance using the GraphPad Prism software, in which x̅̅ means the average value of data, and s denotes the standard deviation. Decline rate of blood glucose: E=[(relative blood glucose of control group - relative blood glucose of drug administration group)/relative blood glucose of control group] × 100%

Note: If there is no significant difference between the relative blood glucose value of the administered group and the relative blood glucose of the control group, the value of E is recorded as 0.

Calculation method of synergistic effect: the determination was made by using Zhengjun Jin's Q value, in which the Q value was obtained by the following formula: Q=E_{a+b}/(Eₐ+E_{b}-Eₐ×E_{b}); whereinE_{a+b} indicated the decline rate of detection indexes of the combination and mixed administration of the two drugs, and Eₐ, E_{b} indicated the decline rate of detection indexes of the two drugs used alone. If Q is between 0.85 and 1.15, it means that the two drugs have an additive effect; Q>1.15, the two drugs have a synergistic effect; Q<0.85, the two drugs have an antagonistic effect.

The results of the experiment are shown in Table 1:

**Table 1 shows the effect of the combination of Alirocumab and semaglutide on blood glucose in hamsters**

| Groups | Animal number (individuals) | Blood glucose (relative concentration) | Blood glucose decline rate E value | Q value |
|---|---|---|---|---|
| Control group | 7 | 0.71±0.049 | | |
| Alirocumab group | 8 | 0.71±0.049 | 0 | |
| Semaglutide group | 7 | 0.61±0.062*^{#} | 14.1% | |
| Alirocumab + Compound 5 group | 8 | 0.51±0.039*^{#$} | 28.2% | 2 |

| | | | | |
|---|---|---|---|---|
| Note : Compared with control group , *P < 0.01 ; Compared with Alirocumab group, ^{#}P<0.01; Compared with Semaglutide group, ^{$}P< 0.01; | | | | |

From the experimental results shown in Table 1, it can be seen that there was no statistically significant difference between the Alirocumab group and the control group, indicating that Alirocumab did not show a hypoglycemic function, and there was a statistically significant difference between the combination-administered group and the control group, indicating that combination-administered group had a significant hypoglycemic function; unexpectedly, the combination-administered group showed statistically significant differences compared to the semaglutide group, indicating that the combination-administered group of the present invention significantly reduced the hamster's blood glucose after administration and that the results of the calculation of the Q-value indicated that the combination of the drugs of the present invention had a synergistic hypoglycemic effect.

### Example 8 Effect of combinations of Evolocumab and compound 5 on blood glucose

### 1. Experimental method

The experimental animals in this Example were wild-type hamsters, and the hamsters were grouped and administered according to the following standard:
1) Control group: subcutaneous injection of excipient samples, subcutaneous injection (administration of 2 µL/kg), once every two days;
2) Evolocumab group: injection of Evolocumab samples (Evolocumab 30 mg/kg) by subcutaneous injection, once every two days;
3) Compound 5 group: injection of compound 5 sample (compound 5: 0.12mg/kg), using subcutaneous injection, once every two days;
4) Evolocumab + Compound 5 group: injection of a pharmaceutical combinations containing Evolocumab and Compound 5 (Evolocumab 30mg/kg, Compound 5: 0.12mg/kg) by subcutaneous injection once every two days.

Sample preparation for each group:
Excipient samples: the excipient samples contained 10 mM sodium acetate, 2.5% proline (w/v) and 0.01% polysorbate 80 (w/v), which was adjusted to pH 5.0.
Evolocumab sample: the excipient contained 10 mM sodium acetate, 2.5% proline (w/v) and 0.01 % polysorbate 80 (w/v), pH=5. Preparation was done by diluting commercially available Repatha^{®} (Evolocumab: 140 mg/mL) with the above mentioned excipient into 15mg/ml, to obtain a 15mg/mL sample of the Evolocumab formulation.
Compound 5 Sample: the excipient contained 7.95 mM disodium hydrogen phosphate, 1.4% propylene glycol (w/v), and 0.55% phenol (w/v), pH=7.3. It was prepared by weighing Compound 5 powder and then adding the excipient to dissolve it into a 0.5 mg/ml solution of Compound 5, and then diluting it with the excipient to form a 0.06 mg/mL sample of Compound 5 solution.
Sample of Evolocumab + Compound 5 pharmaceutical Composition: the excipient contained 10 mM sodium acetate, 2.5% proline (w/v) and 0.01% polysorbate 80 (w/v). The method of preparation was as follows: 1) commercially available Repatha^{®} (Evolocumab: 140 mg/mL) was diluted with the above excipient (pH=5) to form a 30mg/ml solution of Evolocumab, and the Evolocumab solution was adjusted to a pH of 6.75 with sodium hydroxide solution; 2) Compound 5 powder was weighed, and then the above excipient (pH=7.3) was added to form a solution of 0.5 mg/ml of compound 5. Then dilute it into 0.12mg/mL compound 5 solution with the above excipient (pH=7.3), and adjust the pH to 6.75 with acetic acid; 3) Finally, mix the pH-adjusted Evolocumab solution and compound 5 solution in the ratio of 1:1, and then fine-tune the pH to 6.75 after mixing to obtain the pharmaceuticalcomposition comprising 15mg/mL of Evolocumab and 0.06 mg/mL of compound 5, pH=6.75.

On the first day of the experiment, each group of wild-type hamsters was given high-fat and high-sugar feed (containing 10% lard, 10% cholesterol, and 60% fructose) and injected with samples according to the dosing regimen described above for each group, and blood was collected from the inner canthus vein of the hamsters on the day before administration and 7th day after administration, and plasma was anticoagulated with heparin sodium, and plasma was obtained by centrifugation for 10 min at 4°C at 4,000 rpm, and the blood glucose level was measured by glucose assay kit (Biosino Bio-Technology and Science Incorporation.)

### 2. Data processing and experimental results

The obtained blood glucose data were normalized according to the following method: the blood glucose of each group before the administration of the drug was taken as the baseline, the relative concentration of the corresponding time point obtained by comparing the blood glucose at the monitoring point after the administration of the drug with baseline blood glucose , the relative concentration of blood glucose was expressed as x̅̅±s, and the decline rate of the blood glucose of each administration group was calculated by using the analysis of variance using the GraphPad Prism software, in which x̅̅ means the average value of data, and s denotes the standard deviation. Decline rate of blood glucose: E=[(relative blood glucose of control group - relative blood glucose of drug administration group)/relative blood glucose of control group] × 100%

Note: If there is no significant difference between the relative blood glucose value of the administered group and the relative blood glucose of the control group, the value of E is recorded as 0.

Calculation method of synergistic effect: The determination was made by using Zhengjun Jin's Q value, wherein the Q value was obtained by the following formula: Q=E_{a+b}/(Eₐ+E_{b}-Eₐ×E_{b}); in which E_{a+b} indicated the decline rate of monitoring indexes of the combination or mixed administration of the two drugs, and Eₐ, E_{b} indicated the decline rate of monitoring indexes of the two drugs used alone. If Q is between 0.85 and 1.15, it means that the two drugs have an additive effect; Q>1.15, the two drugs have a synergistic effect; Q<0.85, the two drugs have an antagonistic effect.

The results of the experiment are shown in Table 2:

**Table 2 shows the effect of the combination of Evolocumab and compound 5 on blood glucose in hamsters**

| Groups | Animal number (individual) | Blood glucose (relative concentration) | Blood glucose decline rate E value | Q- value |
|---|---|---|---|---|
| Control group | 7 | 0.81±0.066 | | |
| Evolocumab group | 8 | 0.87±0.065 | 0 | |
| Compound 5 group | 7 | 0.57±0.15*^{#} | 30.0% | |
| Evolocumab + Compound 5 group | 8 | 0.41±0.13*^{#$$} | 49.4% | 1.65 |

| | | | | |
|---|---|---|---|---|
| Note : Compared with control group , *P < 0.01 ; Compared with Evolocumab group, ^{#}P<0.01; Compared with Compound 5 group, ^{$$}P< 0.05. | | | | |

From the experimental results shown in Table 2, it can be seen that there was no statistically significant difference between the Evolocumab group and the control group, indicating that Evolocumab did not show a hypoglycemic function, and there was a statistically significant difference between the combination-administered group and the control group, indicating that combination-administered group has a significant hypoglycemic function; unexpectedly, the combination-administered group showed a statistically significant difference in comparison to the Compound 5 group, indicating that the compositions of the present invention can significantly reduce the blood glucose of the hamster after application, and the results of the calculation of the Q-value indicated that the pharmaceutical compositions of the invention have a synergistic hypoglycemic effect.

### Example 9: The effect of Evolocumab in combination with Compound 5 on hyperlipidemia

### 1. Experimental Method

In this example, experimental animals were transgenic mice overexpressing human PCSK9 (4 weeks old). After being fed a high-fat diet for 37 days, their fasting lipid levels were measured. The experimental animals were grouped and dosed as follows:
1) Control group: Subcutaneous injection of vehicle sample (100 µL).
2) Evolocumab group: Subcutaneous injection of Evolocumab sample (Evolocumab: 30 mg/kg).
3) Compound 5 group: Subcutaneous injection of Compound 5 sample (Compound 5: 0.12 mg/kg).
4) Evolocumab + Compound 5 group: Subcutaneous injection of a combination of Compound 5 and Evolocumab (Compound 5: 0.12 mg/kg, Evolocumab: 30 mg/kg).

Sample preparation for each group:
Vehicle sample: Phosphate buffer at pH 6.
Evolocumab sample: Evolocumab (marketed as Repatha^{®}) was dissolved in phosphate buffer at pH 6 to obtain a 7.5 mg/mL Evolocumab sample. Compound 5 sample: Compound 5 was dissolved in phosphate buffer at pH 6 to obtain a 30.75 µg/mL Compound 5 sample.
Evolocumab + Compound 5 pharmaceutical combination sample: Evolocumab and Compound 5 were dissolved in phosphate buffer at pH 6 to obtain a sample containing 7.5 mg/mL Evolocumab and 30.75 µg/mL Compound 5.

On the 5th day after dosing, the lipid levels of the mice were measured. The lipid measurement method was as follows: Blood was collected from the mice via the inner canthus vein, plasma was obtained by centrifuging whole blood at 4°C and 4000 rpm for 10 minutes with sodium heparin as an anticoagulant. LDL-C levels were measured using an LDL-C assay kit (Nanjing Jiancheng Bioengineering Institute).

### 2. Data Processing and Experimental Results

The blood lipid data obtained were normalized using the following method: The blood lipid of each group before administration was taken as baseline, and the relative concentration of lipid at the corresponding time point obtained by comparing the blood lipid at the monitoring points after administration with that before administration. The relative concentration of blood lipid is represented as 'x̅±s', with 'x̅' representing the mean value of the data and 's' representing the standard deviation. Variance analysis was conducted using GraphPad Prism software.

Experimental results are presented in Table 3:

**Table 3: Effect of combination of Evolocumab and Compound 5 on blood lipid levels in hyperlipidemic mice.**

| Group | Number of animals | LDL-C (relative concentration) |
|---|---|---|
| Control group | 3 | 0.67±0.23 |
| Evolocumab group | 3 | 0.25±0.039* |
| Evolocumab + Compound 5 group | 3 | 0.20±0.059* |

| | | |
|---|---|---|
| Note: *P < 0.01, compared to the control group; ^{#}P < 0.01, compared to Evolocumab. | | |

As shown in Table 3, the experimental results indicate that both the Evolocumab group and the combination therapy group had a statistically significant difference compared to the control group. This suggests that both Evolocumab and the combination therapy group demonstrate lipid-lowering effects. Additionally, there was no significant difference between the combination therapy group and the Evolocumab group, indicating that the combination therapy group maintained the lipid-lowering effect of Evolocumab.

In conclusion, as seen from the results of experiments in Examples 7-9, the combination of GLP-1 receptor agonists and PCSK9 inhibitors maintains the lipid-lowering effect of PCSK9 inhibitors. Unexpectedly, these two agents also have a synergistic effect on lowering blood glucose levels.

Example 10: Animal Efficacy Experiment of Evolocumab in Combination with Compound 5

### 1. Experimental Method

In this example, humanized mice overexpressing PCSK9 (B6-hPCSK9) (males, 6 weeks old) were used, totaling 30 mice. These humanized mice were divided into six groups: K0-K5, with 5 mice per group. K0 served as the NC control group (fed a high-fat feed throughout the experiment), while K1-K5 were experimental groups. After a 1-week adaptation period with growth and breeding feed (Keao Xieli Feed), they were fed a high-fat diet for one month (Xietong Pharmaceutical Bio-engineering), and dosing was initiated after the high-fat diet feeding.

The dosing for each experimental group was done via subcutaneous injection once in total, with the dose administrated calculated based on the dose of Evolocumab. The dose in the pre-mix formulation for Evolocumab and Compound 5 was 70 mg: 3 mg. If the dose was doubled (2x), the dose was doubled accordingly. Each group was dosed according to the following plan:
K0: Untreated group, fed growth and breeding feed (Keao Xieli Feed).
K1: Buffer control group, given buffer solution, Evolocumab buffer solution (pH=6.2, 9.0 mg/mL anhydrous disodium hydrogen phosphate, 3.9 mg/mL citric acid monohydrate), with a dose of 4mL/kg.
K2: 1x Single Compound 5 Injection Group (Compound 5 Injection Formulation: Compound 5 Formulation-1 compositions: 2g/l Compound 5, 1.42g/l Anhydrous Sodium Hydrogen Phosphate, 14g/l Propylene Glycol, 5.5g/ Phenol), administered at a dose of 530ug/kg with a dosing volume of 4ml/kg.
K3: 1x Single Evolocumab Group (Evolocumab Formulation: 70mg/ml Evolocumab, 9.0mg/ml Anhydrous Sodium Hydrogen Phosphate, 3.9mg/ml Citric Acid Monohydrate, 25mg/ml Proline, 0.1mg/ml Polysorbate 80), administered at a dose of 12.3mg/kg, 4ml/kg.
K4: 1x Evolocumab + Compound 5 combination group (lyophilized sample, see preparation method in formulation example 20), with a dose of 12.3 mg/kg Evolocumab, administered at 4 ml/kg.
K5: 2x Evolocumab + Compound 5 combination group (lyophilized sample, see preparation method in formulation example 20), with a dose of 24.6 mg/kg Evolocumab, administered at 4 mL/kg.

Throughout the experiment, various indicators were monitored with the following frequencies: for weight monitoring, the weight was measured and recorded before dosing and then every other day after blood collection, this was performed a total of 3 times. For blood glucose, blood was collected via inner canthus veinpuncture before and after dosing, and plasma was obtained using sodium heparin as an anticoagulant followed by centrifugation at 4°C, 4000rpm for 10 minutes. Blood glucose levels were measured using a glucose assay kit (provided by Biosino Bio-Technology and Science Inc). For low-density lipoprotein cholesterol (LDL-C) monitoring, blood was collected from the eye socket before and after dosing (approximately 100 µl per mouse) in clean 1.5 mL centrifuge tubes. After standing at room temperature for 2 hours, the samples were centrifuged at 1200xg for 10 minutes to obtain clear supernatant, which was stored at -80°C for further testing. LDL-C in the serum samples was measured using an LDL-C test kit (Nanjing Jiacheng Bioengineering Institute).

### 2. Experimental Results

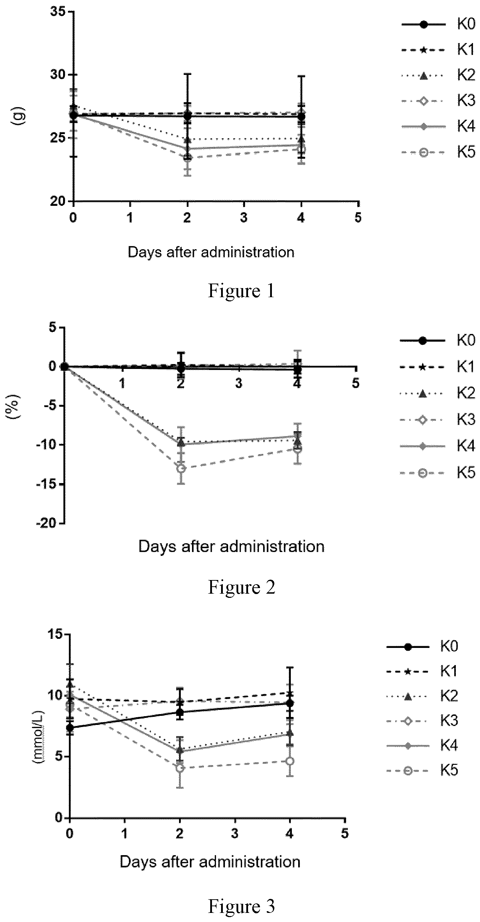

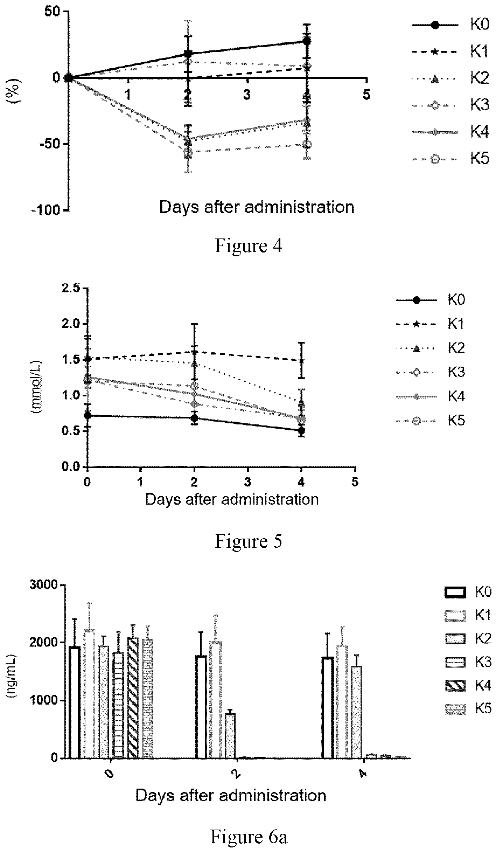

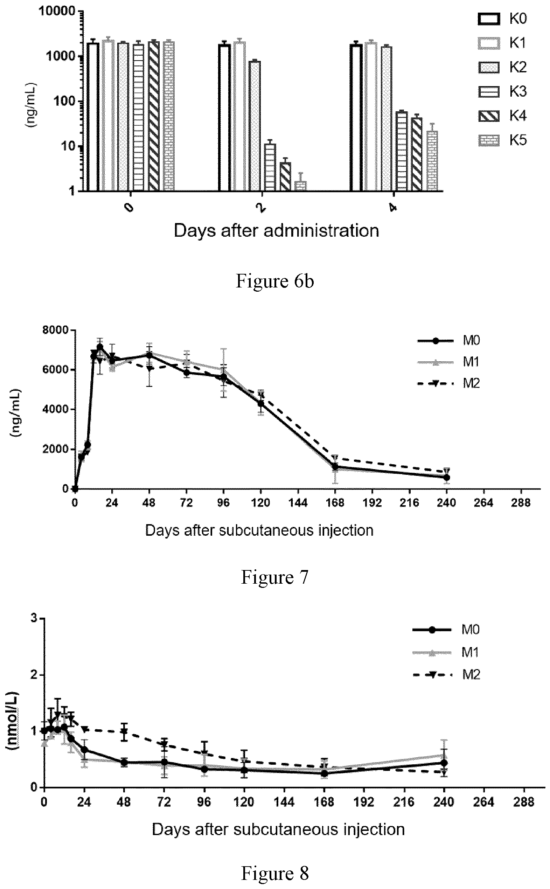

Figures 1-2 show the results of body weight and body weight change rate in humanized mice, while Figures 3-4 present the results of fasting blood glucose and fasting blood glucose change rate in humanized mice. Figure 5 displays the results of LDL-C levels in humanized mice, and Figures 6a-6b illustrate the results of serum free PCSK9.

From the above results,
1) Regarding body weight, Figures 1 and 2 indicate that the body weight of K0 and K1 groups remained unchanged, suggesting that the mice maintained stable conditions during the experiment. The K3 single-dose Evolocumab group did not experience weight loss, indicating no weight reduction effect. However, the remaining three groups, K2, K4, and K5, all showed a significant decrease in body weight. Among them, the body weight change rate of K2 and K4 groups was similar, indicating that the pre-mixed formulation and the single Compound 5 had comparable weight reduction effects. This demonstrates that the pre-mixed formulation of the present invention maintains the original efficacy of Compound 5. The K5 group had a better therapeutic effect than the K4 group, indicating a correlation between the therapeutic effect and the dosage of the pre-mixed formulation.
2) Regarding blood glucose, Figures 3 and 4 show that the blood glucose-lowering effects of the K2 and K4 groups were similar, and the K5 group had a better therapeutic effect than the K2 and K4 groups. This suggests that the Compound 5 in the pre-mixed formulation has a blood glucose-lowering effect similar to that of the single Compound 5 injection solution. The combination of GLP-1 receptor agonists and PCSK-9 antibodies maintains the blood glucose-lowering function of GLP-1 receptor agonists, and there is a correlation between the therapeutic effect and the dosage of the pre-mixed formulation.
3) Regarding LDL-C, Figure 5 demonstrates that the ability to lower LDL-C levels was consistent in the K3, K4, and K5 groups, indicating that the therapeutic effect of Evolocumab in the pre-mixed formulation is similar to that of the single Evolocumab. The combination of GLP-1 receptor agonists and PCSK-9 antibodies maintains the lipid-lowering function of PCSK-9 antibodies.
4) Concerning serum free PCSK9, Figures 6a-6b show that the K3, K4, and K5 groups all exhibited a significant reduction in PCSK9, decreasing serum free PCSK9 from 2000 ng/mL to below 10 ng/mL. Furthermore, the K4 group had a better efficacy than the K3 group, showing a significant difference between them. This indicates that the pre-mixed formulation has a better ability to reduce serum PCSK9 compared to single Evolocumab. Additionally, the K5 group had a better efficacy than the K4 group, demonstrating a dose-dependent effect of the pre-mixed formulation.

In summary, the pre-mixed formulation of Compound 5 and Evolocumab exhibits comparable or higher efficacy compared to their individual use. Example 11: Pharmacokinetics and Pharmacodynamics Experiment of Evolocumab in Combination with Compound 5 in Cynomolgus Monkeys 1. Experimental Method

Six male Cynomolgus monkeys aged 3.9-5.8 years with weights ranging from 3.03 kg to 3.75 kg were selected, with 2 monkeys per group. They were divided into groups and dosed according to the criteria presented in Table 4.

**Table 4: Grouping and dosing methods for Cynomolgus monkeys.**

| Drug Name | Number | Original Antibody Concentration | Administration Route | Dosage |
|---|---|---|---|---|
| Commercial Evolocumab (pH=5.0) (Repatha^{®}) | M0 | 140mg/mL | Administered undiluted, subcutaneous | 2.8mg/k g |
| | | | injection | |
| Evolocumab Formulation (pH=6.2, Formulation: 70mg/ml Evolocumab, 9.0mg/ml anhydrous sodium hydrogen phosphate, 3.9mg/ml citric acid monohydrate, 25mg/ml proline, 0.1mg/ml polysorbate 80) | M1 | 70mg/mL | Administered undiluted, subcutaneous injection | 2.8mg/kg |
| Pre-mixed Compound 5 + Evolocumab Lyophilized Powder [Each vial contains 3mg of Compound 5 + 70mg of Evolocumab] (Prepared as described in Example 20) | M2 | 70mg/vial | Administered without dilution after thorough dissolution in ImL sterile water, subcutaneous injection | 2.8mg/kg |

After the administration, blood samples were collected at the following time points (4h-240h): 4 hours, 8 hours, 12 hours, 16 hours, 24 hours (1 day), 48 hours (2 days), 72 hours (3 days), 96 hours (4 days), 120 hours (5 days), 168 hours (7 days), 240 hours (10 days), 336 hours (14 days), 432 hours (18 days), 528 hours (22 days), and 672 hours (28 days).

### 2. Experimental Results

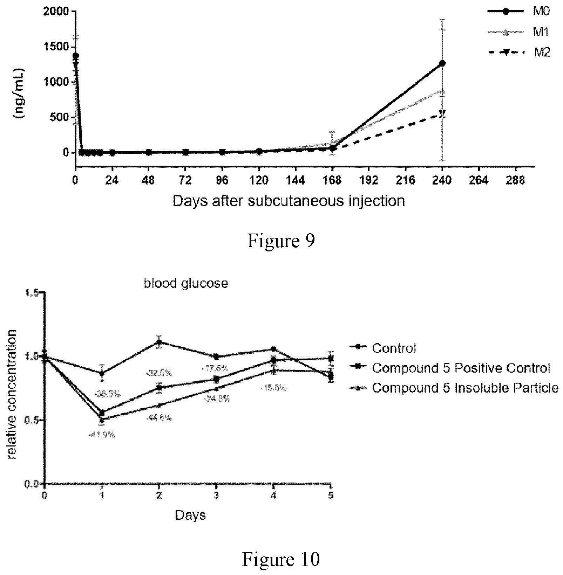

The pharmacokinetic parameter calculation results are shown in Table 5, and the pharmacokinetic (PK) curves are presented in Figure 7. The PK-pharmacodynamic results are displayed in Figures 8-10.

**Table 5: Pharmacokinetic parameters after subcutaneous injection of the compound formulation in Cynomolgus monkeys.**

| Experimental Group | Cmax(mg/L) | Tmax(hr) | T1/2(hr) | AUC0-t (mg/L*hr) | MRT0-t(hr) |
|---|---|---|---|---|---|
| M0 | 7.2 | 16 | 43.1 | 869.2 | 80.0 |
| M1 | 7.05 | 16 | 45.2 | 883.2 | 80.1 |
| M2 | 6.8 | 12 | 56.6 | 908.4 | 85.4 |

From the results presented in Table 5 and Figure 7, there was no significant difference in pharmacokinetics among the Evolocumab Repatha^{®} (M0), the single-component Evolocumab formulation (M1), and the pre-mixed formulation (M2). Specifically, the combination formulation exhibits comparable half-life, AUC0-t, and MRT, etc., compared to that of single component Evolucumab. This indicates that the combination formulation possesses favorable pharmacokinetic properties. Figures 8-9 further demonstrate that the Evolocumab in the combination formulation exhibits efficacy properties consistent with the reference medicine, Repatha^{®}, and single-component Evolocumab.

Examples 12-17 pertain to the pharmaceutical combinations/compositions of the present invention and their preparation methods, as follows:

### Formulation Formulas

### Example 12: Clear Formulation-1

**Table 6: Clear Formulation-1 (1 ml) (pH=6.0)**

| Ingredients | Concentration (mg/ml) |
|---|---|
| Evolocumab | 35 |
| Compound 5 | 0.5 |
| Sodium Hydrogen Phosphate Dihydrate | 1.42 |
| Sucrose | 90 |
| Polysorbate 80 | 0.1 |

### Preparation Process:

(1) Prepare a mixture A by blending disodium hydrogen phosphate dihydrate, sucrose, and Polysorbate 80 according to the specified ratios above. Adjust the pH to 5.0. Prepare a mixture B by blending disodium hydrogen phosphate dihydrate, sucrose, and Polysorbate 80 according to the specified ratios above. Adjust the pH to 7.3.
(2) For Evolocumab, use mixture A to exchange the buffer solution through dialysis and concentrate it to a concentration of 70 mg/mL. Adjust the pH to 6.0 using sodium hydroxide solution, resulting in mixture C.
(3) Weigh the Compound 5 powder and add it to mixture B to dissolve it at 1 mg/mL. Adjust the pH to 6.0 using phosphoric acid, obtaining mixture D.
(4) Mix mixture C and mixture D in a 1:1 ratio to prepare a composite formulation containing 35 mg/mL Evolocumab and 0.5 mg/mL Compound 5 with a pH of 6.0. This is referred to as Clear Formulation-1.

### Example 13: Clear Formulation-2

**Table 7: Clear Formulation-2 (1 ml) (pH=6.5)**

| Ingredients | Concentration(mg/ml) |
|---|---|
| Evolocumab | 35 |
| Compound 5 | 0.5 |
| Sodium Hydrogen Phosphate Dihydrate | 1.42 |
| Sucrose | 90 |
| Polysorbate 80 | 0.1 |

Using steps similar to those in Example 10, a clear composite formulation was obtained, which includes 35 mg/mL of Evolocumab and 0.5 mg/mL of Compound 5, with a pH of 6.5. This formulation is referred to as Clear Formulation-2.

### Example 14: Clear Formulation-3

**Table 8: Clear Formulation-3 (1 ml) (pH=7.0)**

| Ingredients | Concentration(mg/ml) |
|---|---|
| Evolocumab | 35 |
| Compound 5 | 0.5 |
| Sodium Hydrogen Phosphate Dihydrate | 1.42 |
| Sucrose | 90 |
| Polysorbate 80 | 0.1 |

Using steps similar to those in Example 10, a clear composite formulation was obtained, which includes 35 mg/mL of Evolocumab and 0.5 mg/mL of Compound 5, with a pH of 7.0. This formulation is referred to as Clear Formulation-3.

After 30 days of storage, Clear Formulation-3 maintained its clarity upon visual observation. Subsequently, SEC-HPLC was used to detect the formation of Evolocumab aggregates, as follows:

| Sample | Aggregates (%) | Main Peak (%) | Fragments (%) |
|---|---|---|---|
| Clear Formulation-3 | 2.2962 | 97.7038 | 0.0000 |

The high-molecular-weight aggregates formed by Evolocumab in Clear Formulation-3 fell within the mass range (aggregates ≤2.5%), indicating that the Clear Formulation-3 of the present invention exhibits high stability.

### Example 15: Clear Formulation-4

**Table 9: Clear Formulation-4 (1 ml) (pH=7.5)**

| Ingredients | Concentration (mg/mL) |
|---|---|
| Evolocumab | 35 |
| Compound 5 | 0.5 |
| Sodium Hydrogen Phosphate Dihydrate | 1.42 |
| Sucrose | 90 |
| Polysorbate 80 | 0.1 |

The preparation process was the same as in Example 10, resulting in a clear composite formulation containing 35 mg/mL of Evolocumab and 0.5 mg/mL of Compound 5, with a pH of 7.5. This formulation is referred to as Clear Formulation-4.

### Example 16: Clear Formulation-5

**Table 10: Clear Formulation-5 (1 ml) (pH=8.0)**

| Ingredients | Concentration (mg/mL) |
|---|---|
| Evolocumab | 35 |
| Compound 5 | 0.5 |
| Sodium Hydrogen Phosphate Dihydrate | 1.42 |
| Sucrose | 90 |
| Polysorbate 80 | 0.1 |

The preparation process was the same as in Example 10, resulting in a clear composite formulation containing 35 mg/mL of Evolocumab and 0.5 mg/mL of Compound 5, with a pH of 8.0. This formulation is referred to as Clear Formulation-5.

### Example 17: Suspension Formulation-1

**Table 11: Suspension Formulation-1 (1 mL) (pH=5.0)**

| Ingredients | Concentration (mg/mL) |
|---|---|
| Evolocumab | 70 |
| Compound 5 | 0.5 |
| Sodium acetate | 0.82 |
| Proline | 25 |
| Polysorbate 80 | 0.1 |

### Preparation Process:

(1) Prepare a mixture E by combining sodium acetate, proline, and Polysorbate 80 according to the specified quantities. Adjust the pH to 5.0 using acetic acid.
(2) Use commercially available Evolocumab (Repatha^{®}) by exchanging the buffer solution with mixture E through dialysis and concentrating it to a concentration of 140 mg/mL. Adjust the pH to 5.0 using acetic acid, resulting in mixture F.
(3) Weigh the Compound 5 powder and add it to mixture E to dissolve it at 1 mg/mL, obtaining suspension mixture G.
(4) Mix mixture F and mixture G in a 1:1 ratio to prepare a suspension composite formulation containing 70 mg/mL of Evolocumab and 0.5 mg/mL of Compound 5, with a pH of 5.0.

This results in Suspension Formulation-1 containing 70 mg/mL of Evolocumab and 0.5 mg/mL of Compound 5, with a pH of 5.0.

After storing Suspension Formulation-1 for three months, stability data was determined as follows:
(1) Determining the concentration of Evolocumab using Protein A, as detailed in Table 12:

**Table 12: Protein A Detection of Evolocumab Concentration in Suspension Formulation-1.**

| Sample | Theoretical Concentration (mg/mL) | Actual Concentration (mg/mL) |
|---|---|---|
| 4°C Suspension Formulation-1 | 70 | 70.7 |
| Room Temperature Suspension Formulation-1 | 70 | 66.5 |

The concentration of Evolocumab in Suspension Formulation-1 falls within the quality standard range of the originator product (±5%), indicating that the Suspension Formulation-1 of the present invention exhibits high stability.

(2) The formation of Evolocumab aggregates was assessed using SEC-HPLC. The results are detailed in Table 13:

**Table 13: Results of SEC-HPLC Determination of Evolocumab Aggregate in Suspension Formulation-1.**

| Sample | HMW (%) | Main peak (%) | LMW% |
|---|---|---|---|
| 4°C Repatha^{®} (140mg/ml Evolocumab) | 1.461 | 98.469 | 0.070 |
| 4°C Suspension Formulation-1 | 1.566 | 98.353 | 0.081 |
| Room Temperature Suspension Formulation-1 | 1.669 | 98.200 | 0.131 |

The high-molecular-weight aggregates of Evolocumab formed in Suspension Formulation-1 fall within the quality range (aggregates ≤ 2.5%), indicating that the Suspension Formulation-1 of the present invention exhibits high stability.

(3) The acid-to-base peak ratio of Evolocumab in Suspension Formulation-1 was assessed using CEX-HPLC. The results are detailed in Table 14:

**Table 14: CEX-HPLC Assessment of Acid-to-Base Peak Ratio of Evolocumab in Suspension Formulation-1.**

| Sample | Acidic Peak (%) | Main Peak (%) | Alkaline Peak (%) |
|---|---|---|---|
| 4°C Repatha^{®} (140mg/ml Evolocumab) | 26.204 | 64.173 | 9.624 |
| 4°C Suspension Formulation-1 | 25.559 | 64.414 | 10.027 |
| Room Temperature Suspension Formulation-1 | 24.1 | 64.193 | 11.707 |

The acid-to-base peak ratio of Evolocumab in Suspension Formulation-1 falls within the quality range of the original drug (main peak & 51%, basic peak ≤ 16%), demonstrating that Suspension Formulation-1 of the present invention exhibits high stability.

(4) The ratio of the light and heavy chains of Evolocumab was assessed using reducing CE-SDS. The results are provided in Table 15:

**Table 15: Reduced CE-SDS Assessment of the Light and Heavy Chain Ratio of Evolocumab in Suspension Formulation-1.**

| Sample | Pre-LC+LC+HC(%) |
|---|---|
| 4°C Repatha^{®}(140mg/ml Evolocumab) | 98.13% |
| 4°C Suspension Formulation-1 | 98.21% |
| Room Temperature Suspension Formulation-1 | 97.09% |

The ratio of the light and heavy chains of Evolocumab in Suspension Formulation-1 falls within the quality range of the original drug (Pre-LC+LC+HC & 95%), indicating that Suspension Formulation-1 of the present invention possesses high stability.

(5) The purity of Compound 5 was assessed using RPC.

After thoroughly mixing Suspension Formulation-1, 100 µL of the suspension was extracted. After centrifugation at 3000 rpm for 10 minutes, the upper layer containing Evolocumab was removed, leaving the precipitated Compound 5 powder at the bottom. The bottom's Compound 5 powder was then redissolved in a 7.95 mM disodium hydrogen phosphate solution for analysis. The results of the analysis are presented in Table 16:

**Table 16: RPC Analysis of the Purity of Compound 5 in Suspension Formulation-1.**

| Sample | Pre-Peak (%) | Main Peak (%) | Post-Peak (%) |
|---|---|---|---|
| -20°C Compound 5 Standard (Dissolved in 10mM Sodium Dihydrogen Phosphate) | 0.0000 | 99.0003 | 0.9997 |
| 4°C Suspension Formulation-1 | 0.1164 | 99.37 | 0.5135 |
| Room Temperature Suspension Formulation-1 | 0.1979 | 97.8329 | 1.9692 |

The purity of Compound 5 in Suspension Formulation-1 falls within the quality standard range (main peak & 90%).

(6) The activity of Compound 5 was assessed. The results are provided in Table 17:

**Table 17: Activity Assessment of Compound 5 in Suspension Formulation-1.**

| Sample | EC₅₀ (ng/mL) | Relative Potency (%) |
|---|---|---|
| -20°C Compound 5 Standard (Dissolved in 10mM Sodium Dihydrogen Phosphate) | 0.9007 | 100% |
| 4°C Suspension Formulation-1 | 0.6869 | 131% |
| Room Temperature Suspension Formulation-1 | 1.0030 | 90% |

The activity of Compound 5 in Suspension Formulation-1 falls within the quality range (70% ≤ Relative Potency ≤ 130%).

In summary, as observed in (1)-(6), the stability test results for Suspension Formulation-1 comply with all quality standards, indicating that the Suspension Formulation of the present invention possesses high stability. Example 18: Suspension Formulation-2

**Table 18: Suspension Formulation-2 (1 mL) (pH=5.0)**

| Ingredients | Concentration(mg/ml) |
|---|---|
| Evolocumab | 70 |
| Compound 5 | 0.5 |
| Glycerol | 100 µL/mL |
| Sodium acetate | 0.82 |
| Proline | 25 |
| Polysorbate 80 | 0.1 |

### Preparation Process:

(1) Prepare mixture H by combining sodium acetate, proline, and Polysorbate 80 according to the specified quantities above. Adjust the pH to 5.0 using acetic acid. Prepare mixture I (with a glycerol proportion of 20%) by combining glycerol, sodium acetate, proline, and Polysorbate 80 in the specified proportions. Adjust the pH to 5.0 using acetic acid.
(2) Use commercially available Evolocumab (Repatha^{®}) and purify it in the laboratory. Subsequently, exchange the buffer solution with mixture H through dialysis and concentrate it to a concentration of 140 mg/mL. Adjust the pH to 5.0 using acetic acid, resulting in mixture J.
(3) Weigh the Compound 5 powder and add it to mixture I to dissolve it at 1 mg/mL, resulting in suspension mixture K.
(4) Mix mixture J and mixture K in a 1:1 ratio to prepare a suspension composite formulation containing 70 mg/mL of Evolocumab and 0.5 mg/mL of Compound 5, with a pH of 5.0.

This results in Suspension Formulation-2 containing 70 mg/mL of Evolocumab and 0.5 mg/mL of Compound 5, with a pH of 5.0.

After storing Suspension Formulation-2 for one month, stability data was determined as follows.
(1) Determining the concentration of Evolocumab using Protein A detection, as detailed in Table 19:

**Table 19: Protein A Detection of Evolocumab Concentration in Suspension Formulation-2.**

| Sample | Theorectical concentration (mg/mL) | Actual concentration (mg/mL) |
|---|---|---|
| Room Temperature Suspension Formulation-2 (Containing 10% Glycerol) | 70 | 72.6 |

The concentration of Evolocumab monoclonal antibody in Suspension Formulation-2 is within the reference medicine quality standard range (±5%), indicating that the Suspension Formulation of the present invention possesses high stability.

(2) SEC-HPLC detection of Evolocumab monoclonal antibody polymer formation is detailed in Table 20:

**Table 20: SEC-HPLC detection of Evolocumab monoclonal antibody polymer in Suspension Formulation-2**

| Sample | HMW (%) | Main peak (%) | LMW% |
|---|---|---|---|
| 4°C Laboratory-Purified Evolocumab (140mg/ml) | 2.142 | 97.803 | 0.055 |
| Room Temperature Suspension Formulation-2 (Containing 10% Glycerol) | 2.183 | 97.675 | 0.142 |

The aggregates ratio of Evolocumab monoclonal antibody in Suspension Formulation-2 falls within the quality standard range(aggregates ≤2.5%), demonstrating that the Suspension Formulation of the present invention exhibits high stability.

(3) Detection for the acid-base peak ratio of Evolocumab monoclonal antibody in Suspension Formulation-2 by CEX-HPLC is shown in Table 21:

**Table 21: CEX-HPLC detection of the acid-base peak ratio of Evolocumab monoclonal antibody in Suspension Formulation-2**

| Sample | Acidic Peak (%) | Main Peak (%) | Alkaline Peak (%) |
|---|---|---|---|
| 4°C Laboratory-Purified Evolocumab (140mg/ml) | 25.788 | 63.643 | 10.569 |
| Room Temperature Suspension Formulation-2 (Containing 10% Glycerol) | 24.661 | 58.872 | 16.467 |

The Acidic and Alkaline Peak ratio of Evolocumab monoclonal antibody in Suspension Formulation-2 falls within the quality standard range of originator product. (main peak>51%, alkaline peak≤16%).

(4) The results of reduced CE-SDS testing for the light-heavy chain ratio of Evolocumab monoclonal antibody in Suspension Formulation-2 are presented in Table 22:

**Table 22: CE-SDS testing of the light-heavy chain ratio of Evolocumab monoclonal antibody in Suspension Formulation-2.**

| Sample | Pre-LC+LC+HC(%) |
|---|---|
| 4°C Laboratory-Purified Evolocumab (140mg/ml) | 98.79% |
| Room Temperature Suspension Formulation-2 (Containing 10% Glycerol) | 98.92% |

The light-heavy chain ratio of Evolocumab monoclonal antibody in Suspension Formulation-2 falls within the quality standard range of originator product. (Pre-LC+LC+HC≥95%).

(5) The results of RPC testing for the purity of Compound 5 in Suspension Formulation-2 are presented in Table 23:

**Table 23: RPC testing of the purity of Compound 5 in Suspension Formulation-2.**

| Sample | Pre-Peak (%) | Main Peak (%) | Post-Peak (%) |
|---|---|---|---|
| -20°C Compound 5 Standard Solution (Compound 5 Dissolved in 10mM Sodium Dihydrogen Phosphate) | 0.0000 | 99.0003 | 0.9997 |
| Room Temperature Suspension Formulation-2 (Containing 10% Glycerol) | 0.3432 | 98.022 | 1.6347 |

The purity of compound 5 in Suspension Formulation-2 falls within the quality standard range. (Main peak ≥ 90%).

In conclusion, the stability results of Suspension Formulation-2 meet the standards of various testing methods, indicating that the suspension formulation of the present invention exhibits a high level of stability. Example 19 The hypoglycemic effects of Compound 5 in the form of suspension

### 1. Experimental methods

The experiment was conducted using wild-type C57BL6 mice, and the mice were grouped and administered as follows:
1) Control group: Subcutaneous injection of the excipient sample.
2) Compound 5 positive control group: a single subcutaneous injection of Compound 5 in solution state, with a dose of 0.12 mg/kg of Compound 5.
3) Compound 5 insoluble particle group: Subcutaneous single-dose injection of Compound 5 insoluble particle sample, with a dose of 0.12 mg/kg of Compound 5.

The samples for each group were prepared as follows:
Excipient sample: The excipient sample contains 10mM sodium acetate, 2.5% (w/v) proline, 0.01% (w/v) Polysorbate 80, pH 5.0.
Compound 5 positive control sample: The excipient contains 7.95mM disodium hydrogen phosphate, 1.4% glycerol (w/v), 0.55% phenol (w/v), pH 7.3. Preparation method: Compound 5 powder was weighed and added to the excipient above to make a 0.5 mg/ml Compound 5 solution, which was then diluted to 0.06 mg/ml with the excipient.
Compound 5 insoluble particle sample: The excipient contains 10mM sodium acetate, 5% (w/v) proline, 0.01% (w/v) Polysorbate 80, pH=0.5. Preparation method: Compound 5 powder was weighed and added to the excipient above to make a 0.5 mg/ml solution, which was then diluted to 0.06 mg/mL with the excipient.

Blood samples were collected from the inner canthus vein of the mice before dosing, and on the 1st, 2nd, 3rd, 4th, and 5th day post-dosing. Plasma was obtained by centrifuging the blood at 4°C, 4000rpm for 10 minutes, with sodium heparin as an anticoagulant. Glucose levels in the plasma were measured using a glucose measurement kit (Provided by Biosino Bio-Technology And Science Incorporation).

### 2. Experimental results

Compound 5 exists as insoluble particles in the pH 5 vehicle. It is intended to investigate the hypoglycemic effect of Compound 5 in its particle state. The experimental results are shown in Figure 11, and the lowest blood glucose levels were observed on the first day. The obtained blood glucose data were normalized using the following method: blood glucose values at monitoring points after administration were compared to the baseline blood glucose levels for each group, yielding relative concentrations at corresponding time points. Blood glucose relative concentrations are denoted as x̅±s, where x̅ represents the mean value of the data, and s represents the standard deviation. Variance analysis was conducted using GraphPad Prism software, and the hypoglycemic effect on the first day post-administration is as follows:

**Table 24: Validation of the hypoglycemic effects of Compound 5 insoluble particles.**

| Group | Number of Subjects (individuals) | Blood Glucose (Relative Concentration) |
|---|---|---|
| Control group | 6 | 0.87 ± 0.16 |
| Compound 5 Positive Control Group | 6 | 0.56 ± 0.058* |
| Compound 5 Insoluble Particle Group | 6 | 0.50 ± 0.11 * |

| | | |
|---|---|---|
| Note: *P < 0.01, compared to the control group; ^{#}P < 0.01, compared to the Compound 5 positive control group. | | |

The experimental results show that, compared to the Compound 5 positive control group, the hypoglycemic effect of Compound 5 in the form of insoluble particles is consistent with that of the Compound 5 positive control, indicating the feasibility of the suspension formulation.

### Example 20 Lyophilized Formulation-1

**Table 25 Formulation of Lyophilized Formulation-1 before freeze-drying (1mL)**

| Ingredients | Concentration (mg/mL) |
|---|---|
| Evolocumab | 70 |
| Compound 5 | 3 |
| Anhydrous Disodium Hydrogen Phosphate | 9.0 |
| Citric Acid Monohydrate | 3.9 |
| Proline | 25 |
| Polysorbate 80 | 0.1 |

### Preparation Process:

(1) Weigh 8.965g anhydrous disodium hydrogen phosphate, 3.872g citric acid monohydrate, 125g proline, and 0.5g Tween80. Add purified water, stir until dissolved, and make up to a final volume of 1 L to prepare the excipient addition solution.
(2) Take a volume (V) of initial solution (Evolocumab) with a concentration of 81.25-93.75 mg/ml. Add 1/4V volume of the excipient addition solution to prepare a stock solution with a concentration of 70 mg/ml.
(3) Weigh the stock solution and compound 5 powder (The content of compound 5 powder in the final preparation is 3 mg/ml.). Stir in a beaker until dissolved to prepare the Compound 5 liquid formulation. Dispense the prepared liquid formulation into 2R vials at 1 mL per vial.
(4) Freezing: Place the vials in a freeze dryer at -40°C for 3 hours, followed by a 4-hour annealing step at -20°C. Subsequently, freeze the solution again at -40°C.
(5) Drying: When the pressure reaches 0.2mbar, set the primary drying temperature to -24°C for 5 hours. Then, adjust the drying temperature to 27°C for 16 hours until the moisture content of the lyophilized product is less than 2.5%. Store the lyophilized product at -20°C. The 37°C accelerated stability results for the lyophilize formulation are shown in Table 26:

**Table 26 37°C Accelerated Stability Results for lyophilized Formulation 1**

| Sample (37°C Accelerate) | | Day 0 | | | Day 14 | | | Day 30 | | |
|---|---|---|---|---|---|---|---|---|---|---|
| | | SEC | | | SEC | | | SEC | | |
| | | Aggregat es | Protein | Frag ments | Aggreg ation | Protein | Fragme nts | Aggreg ates | Protein | Frag ments |
| Lyophilized Formulation -1 | Evolocumab | 0.956 | 99.015 | 0.029 | 1.718 | 98.256 | 0.026 | 1.094 | 98.881 | 0.025 |
| | Compound 5 | 0.345 | 99.655 | | 0.433 | 99.567 | | 0.351 | 99.649 | |
| Evolocumab formulation-1 | | 1.811 | 98.111 | 0.078 | 2.283 | 97.517 | 0.199 | 3.2 | 96.424 | 0.376 |
| Compound 5 formulation-1 | | 0.655 | 99.345 | | | | | 2.158 | 97.842 | |

| Sample (3 7 °C Accelerate) | | Day 47 | | | Day 60 | | | Day 77 | | |
|---|---|---|---|---|---|---|---|---|---|---|
| | | SEC | | | SEC | | | SEC | | |
| | | Aggregat es | Protein | Frag ments | Aggreg ation | Protein | Fragme nts | Aggreg ates | Protein | Fragme nts |
| Lyophilized Formulation -1 | Evolocumab | | | | 1.203 | 98.745 | 0.052 | | | |
| | Compound 5 | | | | 0.426 | 99.574 | | | | |
| Evolocumab formulation-1 | | 3.438 | 96.102 | 0.46 | | | | 4.264 | 95.057 | 0.679 |
| Compound 5 formulation-1 | | 2.7 | 97.29 | | | | | | | |

For Evolocumab Formulation-1: 140 mg/mL Evolocumab, 1.2 g/L acetic acid, 25 g/L proline, 0.1 g/L Polysorbate 80, pH=5.0. For Compound 5 Formulation-1: 2 g/L compound 5, 1.42 g/L anhydrous disodium hydrogen phosphate, 14 g/L propylene glycol, 5.5 g/L phenol.

The prepared freeze-dried formulations were stored at 37° C for 60 days. The proteins and aggregates of Evolocumab and Compound 5 in the combination formulation remained within the specified quality range (Evolocumab: aggregates <_ 4.0%, main peak ≥ 96%, Compound 5: aggregates <_ 0.8%). This indicates that the lyophilized formulation of the present invention possesses high stability.

The present invention has been described in the above examples. It should be understood that the above examples are provided for illustrative and explanatory purposes, and not intended to limit the scope of the invention to the described examples. Furthermore, those skilled in the art will appreciate that the invention is not limited to the above examples, and that various changes and modifications can be made based on the teachings of the invention, all falling within the scope of protection required by the invention. The scope of protection of the invention is defined by the appended claims and their equivalents.

## Claims

1. A pharmaceutical combination, comprising:
a) a protein drug for alleviating or treating cholesterol-related symptoms, preferably, the protein drug is selected from a PCSK9 inhibitor, an ANGPTL3 inhibitor, an ANGPTL4/8 inhibitor, an Apoc3 inhibitor and an apolipoprotein(a) inhibitor, and further preferably, the protein drug is a PCSK9 inhibitor; and
b) a hypoglycemic polypeptide drug, preferably, the polypeptide drug is selected from a GLP-1 receptor agonist, a GIP receptor agonist, a GCCR receptor agonist, a FGF21 agonist and an agonist simultaneously targeting any two or three targets of GLP-1, GIP, GCCR or FGF21. Further preferably, the polypeptide drug is a GLP-1 receptor agonist.

2. The combination according to claim 1, wherein the PCSK9 inhibitor is an antibody or an antigen-binding fragment thereof that specifically binds to PCSK9.

3. The combination according to any one of claims 1 or 2, wherein the PCSK9 inhibitor is selected from at least one from the group consisting of from the group consisting of alirocumab, evolocumab, Lodelcizumab, Ralpancizumab, Bococizumab, LY3015014, LIB-003, SHR-1209, AK-102, JS-002, SAL-003, AK-102 and ATH-06; preferably the PCSK9 inhibitor is alirocumab or evolocumab.

4. The combination according to any one of claims 1 to 3, wherein the GLP-1 receptor agonist is selected from the group consisting of polyethylene glycol loxenatide, dulaglutide, semaglutide, albiglutide, N-ε²⁶-(17-Carboxyheptadecanoylamino)-4(S)-carboxybutanoyl-[Arg34]GLP-1-(7-37) peptide, N-ε²⁶-(17-carboxyheptadecanoylamino)-4(S)-carboxybutanoyl-[Gly8,Arg34]GLP-1-(7-37) peptide, N-ε²⁶-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl][ Gly8,Arg34]GLP-1-(7-37) peptide, N-ε³⁰-[2-(2-[2-(2-[2-(2-[4-(17-Carboxyheptadecanoylamino)-4(S)-carboxybutanoylamino]ethoxy)ethoxy]acetamido)ethoxy]ethoxy)acetyl]( Val⁸ Glu²² Lys³⁰ Arg^{26,34}-GLP-1(7-37)) peptide, or N-ε²³-[2-(2-[2-(2-[2-(2-[4-(17-Carboxyheptadecanoylamino)-4(S)-carboxybutanoylamino]ethoxy)ethoxy]acetamido)ethoxy]ethoxy)acetyl]( Val⁸ Glu²² Lys²³ Arg^{26,34}-GLP-1(7-37)) peptide, or the GLP-1 receptor agonist is a compound of formula B:
[Acy-(L1)ᵣ-(L2)_{q}]-G1 (B),
wherein G1 is a GLP-1 analogue having Arg and Ala or Gly respectively at positions corresponding to position 34 and position 8, respectively, of GLP-1(7-37) (SEQ ID NO: 1), and [Acy-(L1)ᵣ-(L2)_{q}] is a substituent linked to an ε amino group of the Lys residue at position 26 of the GLP-1 analogue, wherein
r is an integer from 1 to 10, and q is 0 or an integer from 1 to 10;
Acy is a fatty diacid comprising 20-24 carbon atoms, wherein formally, a hydroxyl group has been removed from one of carboxyl groups in the fatty diacid;
L1 is an amino acid residue selected from the following: γGlu, αGlu, βAsp, αAsp, γ-D-Glu, α-D-Glu, β-D-Asp or α-D-Asp;
L2 is a neutral and alkylene glycol-containing amino acid residue;
Acy, L1 and L2 are linked by amide bonds; and
the order of occurrence of L1 and L2 in the formula (B) can be independently interchanged.

5. The combination according to claim 4, wherein,
G1 is [Gly8, Arg34]GLP-1-(7-37) peptide (SEQ ID NO: 2) or [Arg34]GLP-1-(7-37) peptide (SEQ ID NO: 3), and preferably [Gly8, Arg34]GLP-1-(7-37) peptide; and/or
r is 1, 2, 3, 4, 5 or 6; preferably, r is 1, 2, 3 or 4; preferably, r is 1 or 2; preferably, r is 1; and/or
q is 0, 1, 2, 3, 4, 5, 6, 7 or 8; preferably, q is 0, 1, 2, 3 or 4; more preferably, q is 0, 1 or 2; and/or
Acy is a fatty diacid containing 20-23 carbon atoms; preferably, Acy is a fatty diacid containing 20, 21 or 22 carbon atoms.

6. The combination according to any one of claims 4 to 5, wherein,
L2 is -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, - HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably, L2 is -HN-(CH₂)₂-O-(CH₂)₂-O-CH₂-CO-; and/or
L1 is selected from γGlu or βAsp; preferably, L1 is γGlu; and/or
Acy is HOOC-(CH₂)₁₈-CO-, HOOC-(CH₂)₁₉-CO-, HOOC-(CH₂)₂₀-CO-, HOOC-(CH₂)₂₁-CO- or HOOC-(CH₂)₂₂-CO-; preferably, Acy is HOOC-(CH₂)₁₈-CO-, HOOC-(CH₂)₂₀-CO- or HOOC-(CH₂)₂₂-CO-.

7. The combination according to any one of claims 4-6, wherein, the Acy, L1, and L2 in formula (B) are sequentially linked by amide bonds, and the C- terminal of L2 is linked to the ε amino group of the Lys residue at position 26 of the GLP-1 analogue.

8. The combination according to any one of claims 4 to 6, wherein, the GLP-1 receptor agonist is selected from the following compounds:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl] [Gly8, Arg34]GLP-1-(7-37) peptide,
*N*-ε²⁶-(20-carboxyelcosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl] [Arg34]GLP-1-(7-37) peptide,
*N*-ε²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1-(7-37) peptide;
preferably, the compound is selected from the following group consisting of:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Gly8, Arg34]GLP-1-(7-37) peptide, and
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide.

9. The combination according to any one of claims 1-8, wherein, the PCSK9 inhibitor and the GLP-1 receptor agonist are administered simultaneously or separately.

10. The combination according to any one of claims 1-9, wherein, the combination is in the form of a pharmaceutical composition or kit.

11. The combination according to any one of claims 1 to 10, wherein the pharmaceutical combination further comprising one or more pharmaceutically acceptable excipients, preferably, the pharmaceutically acceptable excipients are selected from at least one from the group consisting of buffers, stabilizers, surfactants, and isotonic agents.

12. The pharmaceutical combination according to claim 11, wherein,
the buffer is selected from at least one from the group consisting of citric acid, citrate, sodium acetate, sodium dihydrogen phosphate, glutamate, histidine, disodium hydrogen phosphate, and trishydroxymethylaminomethane buffer, Preferably, the buffer is selected from at least one from the group consisting of citric acid, sodium acetate and disodium hydrogen phosphate; preferably, the buffer is a combination of citric acid and disodium hydrogen phosphate; and/or
the stabilizer is selected from at least one from the group consisting of polyhydroxy hydrocarbons, disaccharides, benzyl alcohol, amino acids, polyols, and phenol, preferably selected from at least one from the group consisting of sucrose, lactose, fructose, maltose, trehalose, sorbitol, mannose, arginine, lysine, methionine, taurine and proline; preferably selected from at least one from the group consisting of histidine, proline and sucrose; and/or
the surfactant is selected from at least one from the group consisting of polysorbate 80, polysorbate 20, and poloxamer 188; and/or
the isotonic agent is selected from at least one from the group consisting of sodium chloride, propylene glycol, and glycerol, preferably propylene glycol.

13. The pharmaceutical combination according to any one of claims 1-12, wherein,
the content of the buffer in the combination is 0.05mM to about 100mM, preferably about 0.05mM to about 85mM, preferably about 0.05mM to about 82mM, preferably about 0.05mM to about 40mM, preferably about 0.1mM to about 30mM, preferably about 1mM to about 20mM, preferably about 5mM to about 15mM, preferably about 5mM to about 10mM; and/or
the content of the stabilizer in the combination is about 0.1% w/v to about 15% w/v, preferably about 0.5% w/v to about 10% w/v, preferably about 2.5% w/v to about 10% w/v; and/or
the content of the surfactant in the combination is about 0.001% w/v to about 10% w/v, preferably about 0.01% w/v to about 1% w/v; and/or
the pH of the pharmaceutical combination is about 4.0-about 8.0, preferably about 4.5-about 7.5, preferably about 4.5-about 7.0, preferably about 4.5-about 6.5, preferably about 5.6-about 6.2, preferably about 4.5-about 5.5, preferably about 5.0 to about 5.5.

14. A pharmaceutical composition, the pharmaceutical composition comprising:
the GLP-1 receptor agonist as defined in any one of claims 1 and 4-8;
the PCSK9 inhibitor as defined in any one of claims 1 and 2-3;
a buffer, wherein the buffer is selected from at least one from the group consisting of citric acid, citrate, sodium acetate, sodium dihydrogen phosphate, glutamate, disodium hydrogen phosphate, and trishydroxymethylaminomethane buffer; preferably the buffer is selected from at least one from the group consisting of citric acid, sodium acetate and disodium hydrogen phosphate; preferably the buffer is a combination of citric acid and disodium hydrogen phosphate;
a stabilizer, wherein the stabilizer is selected from at least one from the group consisting of polyhydroxy hydrocarbons, disaccharides, benzyl alcohol, amino acids, polyols, and phenol, preferably, the stabilizer is selected from at least one from the group consisting of sucrose, lactose, fructose, maltose, trehalose, sorbitol, mannitol, arginine, lysine, methionine, taurine, histidine and proline; preferably, the stabilizer is selected from at least one from the group consisting of histidine, proline and sucrose; and
a surfactant, wherein the surfactant is selected from at least one from the group consisting of polysorbate 80, polysorbate 20, and poloxamer 188.

15. The pharmaceutical composition according to claim 14, wherein,
the GLP-1 receptor agonist is selected from the following compounds: semaglutide,
N-ε²⁶-(17-Carboxyheptadecanoylamino)-4(S)-carboxybutanoyl-[Arg34]GLP-1-(7-37) peptide,
N-ε²⁶-(17-Carboxyheptadecanoylamino)-4(S)-carboxybutanoyl-[Gly8,Arg34]GLP-1-(7-37) peptide,
N-ε²⁶-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl][ Gly8,Arg34]GLP-1-(7-37) peptide,
N-ε³⁰-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val⁸ Glu²² Lys³⁰ Arg^{26,34-}GLP-1(7-37)) peptide,
N-ε²³-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val⁸ Glu²² Lys²³ Arg^{26,34}-GLP-1(7-37)) peptide,
N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[19-Carboxynonadenylamino]-4(S)-carboxybutanoylamino)ethoxy]ethoxy)acetyl] [Gly8, Arg34]GLP-1-(7-37) peptide,
N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[21-Carboxynodecanoylamino]-4(S)-carboxybutanoylamino)ethoxy]ethoxy)acetyl][Gly8, Arg34 ]GLP-1-(7-37) peptide,
N-ε²⁶-[2-(2-[2-(2-[2-(2-[4-(23-Carboxytricanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[23-Carboxytricanoylamino]-4(S)-carboxybutanoylamino)ethoxy]ethoxy)acetyl][Gly8, Arg34 ]GLP-1-(7-37) peptide,
N-ε²⁶-(23-Carboxytricosylamino)-4(S)-carboxybutanoyl-[Gly8, Arg34]GLP-1-(7-37) peptide,
N-ε²⁶-(19-Carboxynonadecanoylamino)-4(S)-carboxybutanoyl-[Gly8, Arg34]GLP-1-(7-37) peptide,
N-ε²⁶-(21-Carboxynodecanoylamino)-4(S)-carboxybutanoyl-[Gly8, Arg34]GLP-1-(7-37) peptide,
N-ε²⁶-[2-(2-[2-(2-[2-(2-[4-(19-Carboxynonadenylamino)-4(S)-carboxybutanoylamino]ethoxy)ethoxy]acetylamino)ethoxy]ethoxy)acetyl ][Arg34]GLP-1-(7-37) peptide,
N-ε²⁶-[2-(2-[2-(4-[19-Carboxynonadecanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(21-carboxynodecanoylamino)-4(S)-carboxybutanoylamino]ethoxy)ethoxy [Arg34]GLP-1-(7-37) peptide,
N-ε²⁶-[2-(2-[2-(4-[21-Carboxynodecanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(23-Carboxytricanoylamino)-4(S)-carboxybutanoylamino]ethoxy)ethoxy [Arg34]GLP-1-(7-37) peptide,
N-ε²⁶-[2-(2-[2-(4-[23-Carboxytricosanoylamino]-4(S)-carboxybutanoylamino)ethoxy]ethoxy)acetyl][Arg34]GLP-1-(7-37)peptide,
N-ε²⁶-(23-Carboxytricosanoylamino)-4(S)-carboxybutanoyl-[Arg34]GLP-1-(7-37) peptide,
N-ε²⁶-(19-Carboxynonadecanoylamino)-4(S)-carboxybutanoyl-[Arg34]GLP-1-(7-37) peptide,
N-ε²⁶-(21-Carboxynodecanoylamino)-4(S)-carboxybutanoyl-[Arg34]GLP-1-(7-37) peptide,
N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[20-Carboxyicosanoylamino]-4(S)-carboxybutanoylamino)ethoxy]ethoxy)acetyl] [Gly8, Arg34]GLP-1-(7-37) peptide,
N-ε²⁶-[2-(2-[2-(2-[2-(2-[4-(22-Carboxydodecanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[22-Carboxydodecanoylamino]-4(S)-carboxybutanoylamino)ethoxy]ethoxy)acetyl][Gly8, Arg34 ]GLP-1-(7-37) peptide,
N-ε²⁶-(20-Carboxyeicosanoylamino)-4(S)-carboxybutanoyl-[Gly8, Arg34]GLP-1-(7-37) peptide,
N-ε²⁶-(22-Carboxydodecanoylamino)-4(S)-carboxybutanoyl-[Gly8, Arg34]GLP-1-(7-37) peptide,
N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Arg34]GLP-1-(7-37) peptide,
N-ε²⁶-[2-(2-[2-(4-[20-Carboxyeicosanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(22-Carboxydodecanoylamino)-4(S)-carboxybutanoylamino]ethoxy)ethoxy [Arg34]GLP-1-(7-37) peptide, N-ε²⁶-[2-(2-[2-(4-[22-Carboxydodecanoylamino]-4(S)-carboxybutanoylamino)ethoxy]ethoxy)acetyl][Arg34]GLP-1-(7-37)peptide,
N-ε²⁶-(20-Carboxyeicosanoylamino)-4(S)-carboxybutanoyl-[Arg34]GLP-1-(7-37) peptide, or
N-ε²⁶-(22-Carboxydocanoylamino)-4(S)-carboxybutanoyl-[Arg34]GLP-1-(7-37) peptide;
Preferably, the compound is selected from the following compounds:
N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide,
N-ε²⁶-(19-Carboxynonadecanoylamino)-4(S)-carboxybutanoyl-[Gly8, Arg34]GLP-1-(7-37) peptide,
N-ε²⁶-(19-Carboxynonadecanoylamino)-4(S)-carboxybutanoyl-[Arg34]GLP-1-(7-37) peptide,
N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide, or
N-ε²⁶-[2-(2-[2-(4-[21-Carboxynodecanoylamino]-4(S)-carboxybutanoylamino)ethoxy]ethoxy)acetyl] [Gly8, Arg34]GLP-1-(7-37) peptide.

16. The pharmaceutical composition according to claim 14 or 15, wherein, the PCSK9 inhibitor is alirocumab or evolocumab.

17. The pharmaceutical composition according to any one of claims 14-16, wherein,
the content of the GLP-1 receptor agonist is about 0.05 mg/ml-about 6.0 mg/ml, 0.05 mg/ml-about 5.0 mg/ml, 0.05 mg/ml-about 4.0 mg/ml, 0.05 mg/ml-about 3.0 mg/ml, 0.1 mg/ml-about 2.0 mg/ml, preferably about 0.1 mg/ml-about 1.0 mg/ml, preferably about 0.5 mg/ml-about 1.0 mg/ml; the content of the PCSK9 inhibitor is about 5 mg/ml-about 420 mg/ml, preferably about 5 mg/ml-about 300 mg/ml, preferably about 5 mg/ml-about 200 mg/ml, preferably about 10 mg/ml-about 150mg/ml, preferably about 35mg/ml-140mg/ml, preferably about 35mg/ml-about 70mg/ml; the content of the buffer is about 0.05mM-about 100mM, preferably about 0.05 mM-about 85 mM, preferably about 0.05 mM-about 82 mM, preferably about 0.05 mM-about 40 mM, preferably about 0.1 mM-about 30 mM, preferably about 1 mM-about 20 mM, preferably about 5 mM-about 15 mM, preferably about 5 mM -about 10 mM;
the content of the stabilizer is about 0.1% w/v-about 15% w/v, preferably about 0.5% w/v-about 10% w/v, preferably about 2.5% w/v-about 10% w /v; and
the content of the surfactant is about 0.001% w/v-about 10% w/v, preferably about 0.01% w/v-about 1% w/v.

18. The pharmaceutical composition according to any one of claims 16-17, wherein,
the pH of the pharmaceutical composition is about 4.0-about 8.0, preferably about 4.5-about 7.5, preferably about 4.5-about 7.0, preferably about 4.5-about 6.5, preferably about 4.5-about 6.2, preferably about 5.6-about 6.2, preferably about 6.0-about 6.2, preferably about 4.5-about 5.5, preferably about 5.0-about 5.5.

19. A pharmaceutical composition, the pharmaceutical composition comprising:
about 0.5-5.0 mg/ml, preferably about 0.5-3.0 mg/ml, preferably about 0.5-2.0 mg/ml, preferably about 0.5-1.0 mg/ml of N-ε²⁶-[2-(2-[2-(2-[2-(2-[4-(21 -carboxynodecanoylamino)-4(S)-carboxybutanoylamino]ethoxy)ethoxy
[Gly]acetylamino)ethoxy]ethoxy)acetyl] [Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl][Gly8, Arg34] GLP-1-(7-37) peptide;
about 35 mg/ml-140 mg/ml, preferably about 35 mg/ml-70 mg/ml of alirocumab or evolocumab;
about 8 mM-about 20 mM, preferably about 8 mM-about 10 mM sodium acetate or disodium hydrogen phosphate;
about 2.0% w/v-about 10% w/v, preferably about 2.5% w/v-about 5.0% w/v of sucrose, histidine or proline;
about 0.01% w/v-about 0.1% w/v of polysorbate 20 or polysorbate 80; and a pH of about 4.5-about 6.5, 4.5-about 6.2, preferably about 5.6-about 6.2, preferably about 6.0-about 6.2, 4.5-about 5.5, preferably about 5.0-about 5.5.

20. A pharmaceutical composition, the pharmaceutical composition comprising:
about 0.5-5.0 mg/ml, preferably about 0.5-3.0 mg/ml, preferably about 0.5-2.0 mg/ml of N-ε²⁶-[2-(2-[2-(2-[2-(2-[4-(21-carboxynodecanoylamino)-4(S)-
carboxybutanoylamino] ethoxy)ethoxy]acetylamino)ethoxy]ethoxy)acetyl ][Gly8, Arg34]GLP-1-(7-37) peptide or N-ε²⁶-[2-(2-[2-(4-[19-Carboxynonadenylamino]-4(S)-
carboxybutanoylamino)ethoxy]ethoxy)acetyl][Gly8, Arg34]GLP-1-(7-37) peptide;
about 35 mg/ml to 140 mg/ml, preferably about 35 mg/ml to 70 mg/ml alirocumab or evolocumab;
about 50 mM-70 mM of disodium hydrogen phosphate and/or about 10 mM-30 mM of citric acid monohydrate, preferably about 60-65 mM of disodium hydrogen phosphate and/or about 15 mM-20 mM of citric acid monohydrate; preferably about 63mM of disodium hydrogen phosphate and/ or about 18 mM citric acid monohydrate;
about 2.0% w/v to about 10% w/v, preferably about 2.5% w/v to about 5.0% w/v sucrose, histidine or proline;
about 0.01% w/v to about 0.1% w/v polysorbate 20 or polysorbate 80; and a pH of about 4.5 to about 7.0, preferably about 4.5 to about 6.5, preferably 4.5 to about 6.2, preferably about 5.6 to about 6.2, preferably about 6.0 to about 6.2.

21. A freeze-dried pharmaceutical composition prepared by the pharmaceutical combination or pharmaceutical composition according to claims 1-20.

22. A kit, comprising:
(1) a first therapeutic agent: comprising the GLP-1 receptor agonist defined in any one of claims 1 and 4-8;
(2) a second therapeutic agent: comprising the PCSK9 inhibitor defined in any one of claims 1 and 2-3.

23. The kit according to claim 22, wherein,
the GLP-1 receptor agonist is selected from the following compounds:
semaglutide, N-ε²⁶-(17-Carboxyheptadecanoylamino)-4(S)-carboxybutanoyl-[Arg34]GLP-1-(7-37) peptide,
N-ε²⁶-(17-Carboxyheptadecanoylamino)-4(S)-carboxybutanoyl-[Gly8,Arg34]GLP-1-(7-37) peptide,
N-ε²⁶-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl][ Gly8,Arg34]GLP-1-(7-37) peptide,
N-ε³⁰-[2-(2-[2-(2-[2-(2-[4-(17-Carboxyheptadecanoylamino)-4(S)-carboxybutanoylamino]ethoxy)ethoxy ]acetamido)ethoxy]ethoxy)acetyl]( Val 8 Glu 22 Lys 30 Arg 26,34 -GLP-1(7-37)) peptide, N-ε²³-[2-(2-[2-(2-[2-(2-[4-(17-Carboxyheptadecanoylamino)-4(S)-
carboxybutanoylamino]ethoxy)ethoxy ]acetamido)ethoxy]ethoxy)acetyl]( Val⁸ Glu²² Lys²³ Arg^{26,34}-GLP-1(7-37)) peptide, N-ε²⁶-[2-(2-[2-(2-[2-(2-[4-(19-Carboxynonadenylamino)-4(S)-
carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[19-Carboxynonadenylamino]-4(S)-carboxybutanoylamino)ethoxy]ethoxy)acetyl] [Gly8, Arg34]GLP-1 -(7-37) peptide,
N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[21-Carboxynodecanoylamino]-4(S)-carboxybutanoylamino)ethoxy]ethoxy)acetyl] [Gly8, Arg34]GLP-1-(7-37) peptide,
N-ε²⁶-[2-(2-[2-(2-[2-(2-[4-(23-Carboxytricanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[23-Carboxytricanoylamino]-4(S)-carboxybutanoylamino)ethoxy]ethoxy)acetyl] [Gly8, Arg34]GLP-1-(7-37) peptide,
N-ε²⁶-(23-Carboxytricosylamino)-4(S)-carboxybutanoyl-[Gly8, Arg34]GLP-1-(7-37) peptide,
N-ε²⁶-(19-Carboxynonadecanoylamino)-4(S)-carboxybutanoyl-[Gly8, Arg34]GLP-1-(7-37) peptide,
N-ε²⁶-(21-Carboxynodecanoylamino)-4(S)-carboxybutanoyl-[Gly8, Arg34]GLP-1-(7-37) peptide,
N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Arg34]GLP-1-(7-37) peptide,
N-ε²⁶-[2-(2-[2-(4-[19-Carboxynonadecanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(21-carboxynodecanoylamino)-4(S)-carboxybutanoylamino]ethoxy)ethoxy [Arg34]GLP-1-(7-37) peptide, N-ε²⁶-[2-(2-[2-(4-[21-Carboxynodecanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(23-Carboxytricanoylamino)-4(S)-carboxybutanoylamino]ethoxy)ethoxy [Arg34]GLP-1-(7-37) peptide,
N-ε²⁶-[2-(2-[2-(4-[23-Carboxytricosanoylamino]-4(S)-carboxybutanoylamino)ethoxy]ethoxy)acetyl][Arg34]GLP-1-(7-37)peptide,
N-ε²⁶-(23-Carboxytricosanoylamino)-4(S)-carboxybutanoyl-[Arg34]GLP-1-(7-37) peptide,
N-ε²⁶-(19-Carboxynonadecanoylamino)-4(S)-carboxybutanoyl-[Arg34]GLP-1-(7-37) peptide,
N-ε²⁶-(21-Carboxynodecanoylamino)-4(S)-carboxybutanoyl-[Arg34]GLP-1-(7-37) peptide,
N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[20-Carboxyicosanoylamino]-4(S)-carboxybutanoylamino)ethoxy]ethoxy)acetyl][Gly8, Arg34]GLP-1-(7-37) peptide,
N-ε²⁶-[2-(2-[2-(2-[2-(2-[4-(22-Carboxydodecanoylamino)-4(S)-carboxybutanoylamino]ethoxy)ethoxy]acetylamino)ethoxy]ethoxy)acetyl ][Gly8, Arg34]GLP-1-(7-37) peptide,
N-ε²⁶-[2-(2-[2-(4-[22-Carboxydodecanoylamino]-4(S)-carboxybutanoylamino)ethoxy]ethoxy)acetyl][Gly8, Arg34]GLP-1-(7-37) peptide,
N-ε²⁶-(20-Carboxyeicosanoylamino)-4(S)-carboxybutanoyl-[Gly8, Arg34]GLP-1-(7-37) peptide,
N-ε²⁶-(22-Carboxydodecanoylamino)-4(S)-carboxybutanoyl-[Gly8, Arg34]GLP-1-(7-37) peptide,
N-ε²⁶-[2-(2-[2-(2-[2-(2-[4-(20-Carboxyeicosanoylamino)-4(S)-carboxybutanoylamino]ethoxy)ethoxy ]acetylamino)ethoxy]ethoxy)acetyl ][Arg34]GLP-1-(7-37) peptide,
N-ε²⁶-[2-(2-[2-(4-[20-Carboxyeicosanoylamino]-4(S)-carboxybutanoylamino)ethoxy]ethoxy)acetyl][Arg34]GLP-1-(7-37)peptide,
N-ε²⁶-[2-(2-[2-(2-[2-(2-[4-(22-Carboxydodecanoylamino)-4(S)-carboxybutanoylamino]ethoxy)ethoxy [Arg34]GLP-1-(7-37) peptide,
N-ε²⁶-[2-(2-[2-(4-[22-Carboxydodecanoylamino]-4(S)-carboxybutanoylamino)ethoxy]ethoxy)acetyl][Arg34]GLP-1-(7-37)peptide,
N-ε²⁶-(20-Carboxyeicosanoylamino)-4(S)-carboxybutanoyl-[Arg34]GLP-1-(7-37) peptide, or
N-ε²⁶-(22-Carboxydocosanoylamino)-4(S)-carboxybutanoyl-[Arg34]GLP-1-(7-37) peptide;
Preferably, the compound is selected from the following compounds:
N-ε²⁶-[2-(2-[2-(2-[2-(2-[4-(19-Carboxynonadenylamino)-4(S)-carboxybutanoylamino]ethoxy)ethoxy ]acetylamino)ethoxy]ethoxy)acetyl ][Gly8, Arg34]GLP-1-(7-37) peptide,
N-ε²⁶-[2-(2-[2-(4-[19-Carboxynonadenylamino]-4(S)-carboxybutanoylamino)ethoxy]ethoxy)acetyl][Gly8, Arg34]GLP-1-(7-37) peptide,
N-ε²⁶-(19-Carboxynonadecanoylamino)-4(S)-carboxybutanoyl-[Gly8, Arg34]GLP-1-(7-37) peptide,
N-ε²⁶-(19-Carboxynonadecanoylamino)-4(S)-carboxybutanoyl-[Arg34]GLP-1-(7-37) peptide,
N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide, or
N-ε²⁶-[2-(2-[2-(4-[21-Carboxynodecanoylamino]-4(S)-carboxybutanoylamino)ethoxy]ethoxy)acetyl][Gly8, Arg34]GLP-1-(7-37) peptide.

24. The kit according to claim 22 or 23, wherein the PCSK9 inhibitor is alirocumab or evolocumab.

25. The kit according to any one of claims 22 to 24, wherein the first therapeutic agent is a freeze-dried powder product.

26. The kit according to any one of claims 22 to 25, wherein the second therapeutic agent further comprises:
a buffer, wherein the buffer is selected from at least one from the group consisting of citric acid monohydrate, citrate, sodium acetate, sodium dihydrogen phosphate, glutamate, disodium hydrogen phosphate, and trishydroxymethylaminomethane buffer, preferably the buffer is selected from at least one from the group consisting of citric acid monohydrate, sodium acetate and disodium hydrogen phosphate;
a stabilizer, wherein the stabilizer is selected from at least one from the group consisting of polyhydroxy hydrocarbons, disaccharides, benzyl alcohol, amino acids, polyols, and phenol, preferably, the stabilizer is selected from at least one from the group consisting of sucrose, lactose, fructose, maltose, trehalose, sorbitol, mannitol, arginine, lysine, methionine, taurine, histidine and proline; preferably the stabilizer is selected from at least one from the group consisting of histidine, proline and sucrose; and
surfactant, the surfactant is selected from at least one from the group consisting of polysorbate 80, polysorbate 20, and poloxamer 188.

27. The kit according to any one of claims 22-26, comprising,
(1) the first therapeutic agent: comprising a GLP-1 receptor agonist in an amount of about 0.05 mg to about 6.0 mg, 0.05 mg to about 5.0 mg, 0.05 mg to about 4.0 mg, 0.05 mg to about 3.0 mg, 0.1 mg to about 2.0 mg, preferably about 0.1 mg to about 1.0 mg, preferably about 0.5 mg to about 10 mg;
(2) the second therapeutic agent: comprising a PCSK9 inhibitor in an amount of about 5 mg/ml to about 420 mg/ml, preferably about 5 mg/ml to about 300 mg/ml, preferably about 5 mg/ml to about 200 mg/ml, preferably about 10 mg /ml-about 150 mg/ml, preferably about 35 mg/ml-140 mg/ml, preferably about 35 mg/ml-about 70 mg/ml;
a buffer with a content of about 0.05mM to about 100mM, 0.05mM to about 85mM, preferably about 0.05mM to about 82mM, 0.05mM to about 40mM, preferably about 0.1mM to about 30mM, preferably about 1mM to about 20mM, preferably about 5mM to about 15mM, preferably about 5mM to about 10mM;
a stabilizer with a content of about 0.1% w/v to about 15% w/v, preferably about 0.5% w/v to about 10% w/v, preferably about 2.5% w/v to about 10% w/v;
a surfactant with a content of about 0.001% w/v to about 10% w/v, preferably about 0.01% w/v to about 1% w/v; and
a pH of about 4.0 to about 8.0, preferably about 4.5 to about 7.5, preferably about 4.5 to about 7.0, preferably about 4.5 to about 6.5, preferably about 5.6 to about 6.2, preferably about 4.5 to about 6.2, further preferably about 4.5 to about 6.0.

28. A kit, comprising:
(1) a first therapeutic agent: about 0.5 mg to about 5.0 mg, about 0.5 mg to about 3.0 mg, about 0.5 mg to about 2.0 mg, preferably about 0.5 mg to about 1.0 mg of N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl] [Gly8, Arg34]GLP-1 -(7-37) peptide;
(2) a second therapeutic agent: about 35 mg/ml to 140 mg/ml, preferably about 35 mg/ml to 70 mg/ml of alirocumab or evolocumab;
about 8mM to about 20mM, preferably about 8 mM to about 10 mM of sodium acetate or disodium hydrogen phosphate;
about 2.0% w/v to about 10% w/v, preferably about 2.5% w/v to about 5.0% w/v of sucrose, histidine or proline;
about 0.01% w/v to about 0.1% w/v of polysorbate 20 or polysorbate 80; and
about 4.5 to about 6.5, 4.5 to about 6.2, 4.5 to about 6.0, preferably about 5.0 to about 5.5 of pH.

29. A kit comprising:
(1)a first therapeutic agent: about 0.5 mg to about 5.0 mg, about 0.5 mg to about 3.0 mg, about 0.5 mg to about 2.0 mg, or preferably about 0.5 mg to about 1.0 mg of N-ε²⁶-[2-(2-[2-(2-[2-(2-[4-(21-carboxynodecanoylamino)-4(S)-carboxybutanoylamino]ethoxy)ethoxy]acetylamino)ethoxy]ethoxy)acetyl ][Gly8, Arg34]GLP-1-(7-37) peptide or N-ε²⁶-[2-(2-[2-(4-[19-Carboxynonadenylamino]-4(S)-carboxybutanoylamino)ethoxy]ethoxy)acetyl] [Gly8, Arg34]GLP-1 -(7-37) peptide;
(2) a second therapeutic agent: about 35 mg/ml-140 mg/ml, preferably about 35 mg/ml-70 mg/ml of alirocumab or evolocumab;
about 50 mM-70 Mm of disodium hydrogen phosphate and/or about 10 mM-30 mM of citric acid monohydrate, preferably about 60-65 mM of disodium hydrogen phosphate and/or about 15 mM-20 mM of citric acid monohydrate; preferably about 63mM of disodium hydrogen phosphate and /or about 18mM of citric acid monohydrate;
about 2.0% w/v to about 10% w/v, preferably about 2.5% w/v to about 5.0% w/v of sucrose, histidine or proline;
about 0.01% w/v to about 0.1% w/v of polysorbate 20 or polysorbate 80; and
about 4.5 to about 7.0, preferably about 4.5 to about 6.5, preferably about 4.5 to about 6.2, preferably about 4.5 to about 6.0, preferably about 5.6 to about 6.2, preferably about 5.0 to about 5.5 of pH.

30. Use of the pharmaceutical combination, pharmaceutical composition or kit of any one of claims 1 to 29 in the preparation of a medicament for the treatment of hyperglycemia, diabetes, obesity and/or cholesterol-related diseases.

31. The pharmaceutical combination, pharmaceutical composition or kit of any one of claims 1-29 for use in the treatment of hyperglycemia, diabetes, obesity and/or cholesterol related diseases.

32. A method for treating hyperglycemia, diabetes, obesity and/or cholesterol-related diseases, comprising administering a therapeutically effective amount of the pharmaceutical combination, the pharmaceutical composition or the kit of any one of claims 1-29.
